# EUROPÄISCHE PATENTANMELDUNG

(11) **EP 2 560 016 A1**
(43) Veröffentlichungstag der Anmeldung: **20.02.2013**
(21) Anmeldenummer: 11177908.8
(22) Anmeldetag: 18.08.2011
(51) Int. Cl.: G01R 33/34

(54) **MRT-Lokalspulenvorrichtung für Diagnostik und Intervention**

(71) Anmelder: Lumiani, Agron, 33617 Bielefeld (DE)
(72) Erfinder: Lumiani, Agron, 33617 Bielefeld (DE)
(74) Vertreter: Wittmann, Günther

(57) **Zusammenfassung**

Die Erfindung offenbart eine MRT-Lokalspulenvorrichtung (1), mit
- zumindest einem Träger (T1,T2); und
- einer Mehrzahl von Antennenelementen (K11-K202), die je zum Senden und/oder Empfangen von MRT-Signalen ausgebildet sind;

wobei die Mehrzahl von Antennenelementen (K11-K202) in dem Träger (T1,T2) angeordnet ist;
dadurch gekennzeichnet, dass der Träger (T1) zumindest eine Interventionsöffnung (2) aufweist, die dazu ausgebildet ist, einen Zugang für ein medizinisches Instrument zum Patienten bereitzustellen. Die MRT-Lokalspulenvorrichtung (1) kann in eine Diagnose-Lokalspulenvorrichtung oder eine Interventions-Lokalspulenvorrichtung und umgekehrt umgewandelt werden, indem ein erster Trägerbereichs (T1) mit Antennenelementen (K11-K23) gegenüber einem zweiten Trägerbereich (T2) mit Antennenelementen (K100-K203) bewegt wird.

## Beschreibung

Die Erfindung betrifft eine MRT-Lokalspulenvorrichtung (HF-Spulenvorrichtung) mit einer Interventionsöffnung.

Ein Magnetresonanzsystem weist einen Magneten auf, der ein statisches Magnetfeld entlang des Patienten in longitudinaler Richtung erzeugt. Eine Gradientenspule bewirkt, dass das statische Magnetfeld entlang der longitudinalen Richtung seinen Wert verändert. Die Resonanzfrequenz der Wasserstoffatome hängt von der Stärke des statischen Magnetfeldes ab. Mittels einer sogenannten Birdcageantenne kann der Spin der Wasserstoffkerne angeregt werden. Aufgrund des sich entlang der Patientenachse ändernden statischen Magnetfeldes weisen die Wasserstoffkerne an unterschiedlichen Orten unterschiedliche Resonanzfrequenzen (Larmorfrequenz) auf. Die Relaxation der Wasserstoffkerne nach der Anregung kann mittels einer Lokalspulenvorrichtung, die an dem Pateinten angeordnet ist, ermittelt werden. Somit kann die Dichte des Gewebes bzw. die Ansammlung von Wasserstoffatomen in einem Patienten ermittelt werden und zur Bildgebung verwendet werden. Zum Erzielen möglichst guter Bilder, soll die Lokalspulenvorrichtung möglichst nahe an der zu untersuchenden Körperpartie angeordnet sein.

Die Funktionsweise von Magnetresonanzsystemen ist dem Fachmann bekannt und unter anderem in Imaging Systems for Medical Diagnostics, Arnulf Oppelt, Publicis Corporate Publishing, ISBN 3-89578-226-2 beschrieben.

MRT-Spulenvorrichtungen umfassen Antennenelemente als Empfangsspulen. Mit diesen Antennenelementen werden HF-Signale von der untersuchten Körperpartie nach der Anregung empfangen. Die MRT-Lokalspulenvorrichtung kann eine sogenannte Lokalspule sein. Es gibt mehrere Ansätze, um das HF-Signal möglichst nahe an die zu untersuchenden Stelle des Körpers zu bringen, um eine gute Signalstärke und mithin auch gute und aussagekräftige Befunde zu erhalten.

Zum einen können sich Antennenelemente in einem starren Träger einer Lokalspulenvorrichtung befinden, der auf dem Untersuchungstisch angeordnet ist. Die Lokalspulenelemente können alternativ oder zusätzlich in dem Untersuchungstisch integriert sein. Diese Lokalspulenvorrichtung kann für eine Untersuchung der Lendenwirbelsäule und der Brustwirbelsäule sowie für Ganzkörperuntersuchungen verwendet werden. Bei der Untersuchung der Prostata liegt der Patient im Stand der Technik auf dem Rücken. Die zuvor genannte starre Lokalspulenvorrichtung befindet sich am Rücken des Patienten.

Ferner sind zylinderförmige nicht angepasste starre Lokalspulenvorrichtungen bekannt (beispielsweise Schädelspule, Kniespule). Es sind auch flexible an die Körperform angepasste Lokalspulenanordnungen bekannt. Beispielsweise sind auch Spulenvorrichtungen in Manschettenform bekannt, die flexibel genug sind, so dass sie um die zu untersuchende Körperpartie herum gelegt werden können (beispielsweise Schulter-, Handgelenk-, Abdomen- oder Beckenspulen). Die Lokalspulen können auch Anregungssignale senden.

Das MRT-Verfahren wird zur Diagnostik eingesetzt, um Befunde zu erhalten. Zur Untersuchung der Prostata wird bei MRT-Systemen mit einem statischen Magnetfeld mit einer Stärke bis etwa 1,5 T eine Bauchlokalspulenvorrichtung, eine Rückenlokalspulenvorrichtung und eine Endorektallokalspule verwendet. Die Endorektallokalspule wird mittels Luft im Dickdarm des Patienten fixiert. Die Luft verursacht unerwünschte Artefakte im MRT-Bild, insbesondere bei hochempfindlichen Sequenzen (EPI-Diffusionssequenz). Aus diesem Grund wird bei MRT-Systemen mit einem statischen Magnetfeld von etwa 3 T oder mehr meistens keine Endorektallokalspule eingesetzt, da andernfalls zusätzliche Artefakte auftreten.

Andererseits gibt es MRT-gestützte Therapie- und Diagnoseverfahren, bei denen vor einer Behandlung immer wieder MRT-Aufnahmen gemacht werden, um den exakten Verlauf der entsprechenden Behandlung zu überwachen (z. B. MRTkontrollierte HIFU-Therapie der Prostata).

Ein weiteres Beispiel ist die MRT-gestützte rektale Biopsie durch den Dickdarm zur Diagnose des Prostatakarzinoms. Bei diesem Verfahren besteht die Gefahr, dass Bakterien aus dem Dickdarm in die Prostata gelangen können. Ein weiterer Nachteil dieses Verfahrens ist, dass es durch einen Computer gesteuert automatisch abläuft. Somit ist die Biopsie der Kontrolle des Arztes entzogen, was die Treffsicherheit der Biopsie reduzieren kann, wenn sich der Patient bewegt. Bei diesem Verfahren ist darüber hinaus keine lokale Anästhesie möglich.

Der Erfindung liegt daher die Aufgabe zugrunde, eine MRT-Lokalspulenvorrichtung zu schaffen, durch die der Einsatzbereich entsprechender MRT-Spulenvorrichtungen erweitert wird.

Diese Aufgabe wird durch eine MRT-Lokalspulenvorrichtung mit den Merkmalen des Anspruchs 1 gelöst. Vorteilhafte Ausführungsformen finden sich in den Unteransprüchen.

Erfindungsgemäß weist die MRT-Lokalspulenvorrichtung (HF-Spule) einen Träger und eine Mehrzahl von Antennenelementen auf, die je zum Empfangen von MRT-Signalen und/oder zum Senden von Anregungssignalen ausgebildet sind. Die Mehrzahl von Antennenelementen ist in dem Träger angeordnet. Der Träger weist zumindest eine Interventionsöffnung auf, die dazu ausgebildet ist, einen Zugang für ein medizinisches Instrument zum Patienten bereitzustellen. Das medizinische Instrument kann durch die Interventionsöffnung zum Patienten geführt werden. Bevorzugt sind die Antennenelemente in unmittelbarer Nähe der Interventionsöffnung positioniert, so dass der Empfang von MRT-Signalen nahe dem bzw. über dem zu untersuchenden Organ (beispielsweise über der Prostata) des Patienten erfolgen kann. Gleichzeitig wird im Bereich der Interventionsöffnung ein Zugangsweg zur Punktion, Intervention oder minimal invasiven Therapie geschaffen. So können etwa MRT-Bilder erzeugt werden, um ein Instrument - wie z. B. die Nadelspitze einer Punktionskanüle - relativ zu einer Läsion zu lokalisieren und Probeentnahmen aus der Läsion bzw. aus einem Organ durchzuführen. Die Behandlungsdauer kann dadurch deutlich reduziert werden. Auch anderweitige MRT-gesteuerte Interventionen sowie minimal invasive Therapien sind durchführbar sowie kontrollierbar. Somit wird durch die Erfindung eine multifunktionale Lokalspulenvorrichtung für verschiedene Anwendungsbereiche, beispielsweise für den Bereich der Diagnose, den Bereich der Intervention und den Bereich der Therapie, geschaffen.

Der Ausdruck Interventionsöffnung ist so zu verstehen, dass sie einen Zugang zum menschlichen oder tierischen Körper zur Diagnose und/oder zur Therapie bereitstellt. Die Interventionsöffnung kann auch als Instrumentzugangsöffnung bezeichnet werden. Die Interventionsöffnung kann auch zum Vorbereiten einer Operation dienen.

Die Antennenelemente können als Spulenelemente ausgebildet sein. Die Antennenelemente werden in der Praxis auch als Kanäle bezeichnet. Jeder Kanal kann zumindest einem Antennenelement zugeordnet sein.

Es kann eine höhere Anzahl von Antennenelementen in einem Bereich angeordnet sein, der sich nahe bzw. über einem zu diagnostizierenden Organ befindet. Bei einer Lokalspulenvorrichtung für die Diagnose der Prostata kann eine höhere Anzahl von Antennenelementen in einem Bereich angeordnet sein, der sich nahe bzw. über der Mitte des kleinen Beckens befindet. Ferner kann eine höhere Anzahl von Antennenelementen am großen Becken, insbesondere am Rand (der Lateralwand) des großen Beckens, angeordnet sein, um eventuelle Lymphknotenmetastasen und Knochenmetastasen erkennen zu können. Die Antennenelemente können innerhalb der Lokalspulenvorrichtung inhomogen, beispielsweise nicht äquidistant, angeordnet sein. Der Anmelder behält sich vor, diesen Aspekt separat mit einem Schutzbegehren zu beanspruchen, beispielsweise in Form einer Teilanmeldung.

Der Träger kann als Manschette ausgebildet sein, die öffenbar und schließbar ist.

Insbesondere eignet sich die erfindungsgemäße MRT-Lokalspulenvorrichtung für MRT-gesteuerte Biopsien von kleinen Befunden eines Organs oder einer Region in Millimetergröße, bei schwierigen Zugangswegen, schwierigen Eingriffen und die Vorbereitung eines solchen (z. B. Prostata ohne rektalen Zugang). Dazu ist die Erfindung vorteilhafterweise mit einem Rahmen ausgestattet, der die fensterartige Interventionsöffnung begrenzt. Dieser Rahmen ist bevorzugt so gestaltet, dass er eine Mehrzahl Funktionen beinhaltet. Zum Einen dient er als Einsatz für ein Referenzelement. Dieses wird eingesetzt, um die Position eines Befundes relativ zur Oberfläche festlegen zu können. Bevorzugt ist als Referenzelement ein Gitter aus Röhren, in die eine im MRT-Bild gut sichtbare Flüssigkeit eingekapselt ist, beispielsweise Fischöl. Das Referenzelement kann erste Justagemittel, beispielsweise ein Gitter, aufweisen.

An zumindest zwei nebeneinander liegenden Seiten des Rahmens sind geneigte Gleitflächen vorgesehen, wobei die Gleitflächen gegenüber einer zur Interventionsöffnung senkrechten Achse in die gleiche Richtung geneigt sind. Die Gleitflächen dienen dazu, dass das Referenzelement, welches eine im Wesentlichen der Form der Interventionsöffnung entsprechende Form, bevorzugt eine rechteckige Form, aufweist, gegen eine Ecke der Interventionsöffnung drückt, so dass der Referenzpunkt nicht während der Messungen verschoben werden kann. Durch Messung der Lage des Befundes zum Referenzelement kann dann für nachfolgende Behandlungsschritte die Einstichstelle auf der Hautoberfläche und der Einstichwinkel für eine - insbesondere MR-kompatible - Punktionskanüle festgelegt werden. Da die Interventionsöffnung bzw. der Rahmen der Lokalspulenvorrichtung während der gesamten Behandlung fest bleiben, bleibt auch die Position der Einstichstelle und der Winkel relativ zur Interventionsöffnung bzw. zum Rahmen fest. Die Punktionsnadel oder auch ein anderes Instrument braucht für die nachfolgenden Behandlungs- oder Diagnoseschritte also nur noch in einer Position relativ zur Interventionsöffnung bzw. zum Rahmen ausgerichtet werden.

Die MRT-Lokalspulenvorrichtung kann Anordnungsmittel für ein medizinisches Instrument aufweisen. Daher kann mittels der MRT-Bildgebung eine Körperpartie genau untersucht werden und eine optimale Einsatzposition für ein medizinisches Instrument ermittelt werden. Ferner kann das medizinische Instrument genau zur ermittelten optimalen Einsatzposition geführt werden, da die Position der MRT-Lokalspulenvorrichtung eine exakte Referenzposition liefert.

Für den Interventionsvorgang, beispielsweise bei einer Biopsie, ist erfindungsgemäß eine Nadelhalterung vorgesehen, welche sich relativ zum Rahmen bzw. zur Interventionsöffnung einstellen lässt. Dazu ist die Nadel in der Halterung, bevorzugt in lediglich einer Ebene, schwenkbar und in jeder beliebigen Winkelstellung fixierbar. Die Halterung selbst ist zudem im Bereich der Interventionsöffnung bevorzugt senkrecht zur Schwenkebene verschiebbar.

MRT-Lokalspulenvorrichtung kann Traversenanordnungsmittel umfassen, die zum Anordnen einer in wenigstens einer ersten Richtung verschiebbaren Traverse über der Interventionsöffnung vorgesehen ist. Bevorzugt wird die Halterung an einer die Interventionsöffnung überspannenden Traverse geführt und kann an dieser verschoben werden. Der Rahmen und/oder die Traverse können zweite Justagemittel, beispielsweise Skalen (z. B. Millimeter- oder Zollskalen), aufweisen, um eine genaue Positionierung der Traverse zum Rahmen und der Nadelhalterung zur Traverse zu ermöglichen. Außerdem können die Skalen oder Markierungen als Lineal verwendet werden, um etwa eine Einstichstelle für die Punktion, deren Koordinaten relativ zum Rahmen oder der Interventionsöffnung aus MRT-Aufnahmen ermittelt werden, auf dem Patienten zu markieren. Zudem ist bevorzugt vorgesehen, dass die Traverse selbst am Rahmen verschiebbar und fixierbar geführt wird, so dass mit der Nadelspitze im Wesentlichen jeder Punkt im Bereich der Interventionsöffnung angefahren werden kann. Das Instrument, beispielsweise die Nadel, ist in einer Halterung verschiebbar geführt und kann auch in jeder Position fixiert werden, so dass die Halterung bei der Punktion als Führung oder Unterstützung wirkt.

Mit der Traverse kann ein Schlitten gekoppelt sein, der in einer zweiten Richtung beweglich ist. An der Interventionsöffnung, an der Traverse und/oder am Schlitten kann ein medizinisches Instrument, beispielsweise ein Nadel, angeordnet werden.

Mittels der ersten Justagemittel am Referenzelement kann bei der Bildgebung eine Stellung für ein medizinisches Instrument ermittelt werden. Die Stellung kann eine (optimale) Einsatzstellung des medizinischen Instrumentes sein, beispielsweise die Einstichposition einer Nadel. Mittels der zweiten Justagemittel an der Traverse, am Schlitten, an einer Instrumenthalterung für ein medizinisches Instrument und/oder an der Interventionsöffnung wird das medizinische Instrument zur Einsatzstellung geführt, beispielsweise zur Einstichposition einer Nadel. Der Ausdruck Stellung umfasst räumliche (Orts-)Koordinaten und/oder Winkel.

Zumindest ein Antennenelement kann zwischen zwei Interventionsöffnungen angeordnet sein.

Die Wicklung des zumindest einen Antennenelementes kann im Wesentlichen rechteckig ausgebildet sein. Der Ausdruck im Wesentlichen rechteckig ist so zu interpretieren, dass jede Wicklung eine Abrundung an den Kanten aufweisen kann. Bei einer Ausführungsform ist die Länge des Antennenelementes größer als die Bereite des Antennenelementes. Zumindest ein Antennenelement kann rechtwinklig oder parallel zu einer Kante der Interventionsöffnung angeordnet sein.

Der Träger kann sich in Längsrichtung weiter erstrecken als in Querrichtung. Die Querrichtung des Trägers kann der Längsrichtung (Achse von Kopf zu Füßen) des Patienten entsprechen. Diese Richtung wird auch als vertikale Richtung des Patienten bezeichnet. Zumindest ein Antennenelement kann sich in Querrichtung des Trägers neben der Interventionsöffnung und zumindest ein Antennenelement kann sich in Längsrichtung des Trägers neben der Interventionsöffnung befinden.

Zumindest drei Antennenelemente können in Querrichtung des Trägers neben der zumindest einen Interventionsöffnung angeordnet sein.

Der Träger kann einen ersten Trägerbereich, der dazu ausgebildet ist, an einer ersten Seite einer Körperpartie eines Patienten angeordnet zu werden, und einen zweiten Trägerbereich aufweisen, der dazu ausgebildet ist, an einer zweiten Seite der Körperpartie angeordnet zu werden. Die zumindest eine Interventionsöffnung kann im ersten Trägerbereich ausgebildet sein. Sowohl im ersten Trägerbereich als auch im zweiten Trägerbereich können Antennenelemente angeordnet sein. Der erster Trägerbereich und der zweite Trägerbereich können voneinander gelöst werden. Die erste Seite und die zweite Seite können sich an entgegengesetzten Seiten einer Körperpartie befinden.

Der erste Trägerbereich kann an einer Seite angeordnet sein, an der medizinisch ein Zugang zur Intervention eines Organs möglich ist. Der zweite Trägerbereich kann sich an der dem ersten Trägerbereich entgegengesetzten Seite des Patienten befinden. Mit dieser Ausgestaltung kann ein beliebiges Organ interveniert werden, beispielsweise die Leber, die Bauchspeicheldrüse, die Niere etc.

Der erste Trägerbereich kann dazu ausgebildet sein, auf dem Rücken eines Patienten angeordnet zu werden. Der zweite Trägerbereich kann dazu ausgebildet sein, auf dem Bauch eines Patienten angeordnet zu werden. Der Patient kann während der Bildgebung auf dem Bauch liegen. Diese Ausgestaltung kann zur Intervention der Prostata verwendet werden.

Der erste Trägerbereich kann gegenüber dem zweiten Trägerbereich in zwei unterschiedlichen Stellungen anordenbar sein. Dadurch kann die MRT-Lokalspulenvorrichtung einerseits für die Bildgebung und andererseits für die MRT-gestützte Intervention optimiert werden.

Der erste Trägerbereich kann gegenüber dem zweiten Trägerbereich so beweglich sein, dass die Interventionsöffnung an zwei unterschiedlichen Stellungen anordenbar ist. Der erste Trägerbereich kann gegenüber dem zweiten Trägerbereich so beweglich sein, dass die Interventionsöffnung an zwei unterschiedlichen Stellungen in Querrichtung der der MRT-Lokalspulenvorrichtung und/oder in Longitudinalrichtung des Patienten anordenbar ist.

Die MRT-Lokalspulenvorrichtung kann am Becken eines Patienten anordenbar sein, wobei der zweite Trägerbereich am Rücken (dorsal) des auf dem Rücken liegenden Patienten und der erste Trägerbereich vorne (ventral) am Patienten anordenbar ist. Der erste Trägerbereich so gegenüber dem zweiten Trägerbereich bewegbar ist, dass die Interventionsöffnung an zwei unterschiedlichen Stellungen in longitudinaler Richtung des Patienten anordenbar ist. Die Interventionsöffnung kann sich bei einer Diagnosestellung (bzw. Diagnosikstellung) in longitudinaler Richtung (cranio-caudaler Richtung) über der Prostata befinden. In diesem Fall befindet sich eine Mehrzahl von Antennenelemente in sagittaler Richtung über der Prostata. Bei der Intervention liegt der Patient auf dem Bauch, der erste Trägerbereich befindet sich am Rücken des Patienten (dorsal) und der zweite Trägerbereich befindet sich vorne (ventral) am Patienten. Bei der Interventionsstellung der MRT-Vorrichtung befindet sich die mindestens eine Interventionsöffnung in Körperlängsrichtung (longitudinale Richtung) des Patienten etwa auf der gleichen Höhe wie die Prostata. Folglich befindet sich das die zumindest eine Interventionsöffnung umschreibende Rechteck in sagittaler Richtung über der Prostata. In dieser Stellung ist eine Intervention in die Prostata, beispielsweise eine Punktion, möglich.

Der Patient liegt während der Diagnose (bzw. Diagnostik) auf dem Rücken, da die MRT-Bildgebung bis zu einer Stunde beanspruchen kann. Der Patient soll sich während der MRT-Bildgebung nicht bewegen. Dies kann in der Rückenlage besser gewährleistet werden. Da sich bei der Diagnosestellung des ersten Trägerbereichs mehr Spulen in der Nähe des kleinen Beckens befinden, kann eine höhere Auflösung erzielt werden als in der Interventionsstellung. Bei der Diagnose muss eine höhere Auflösung vorhanden sein, damit beurteilt werden kann, ob eine Läsion, ein Tumor etc. vorhanden ist und wo sich die Läsion, der Tumor etc. befindet. Bei der Intervention kann eine niedrigere Auflösung ausreichen, das die Position der Läsion, des Tumors etc. bekannt ist und mittels der Bildgebung lediglich das Instrument zur Läsion bzw. zum Tumor geführt werden muss.

Die Erfindung betrifft auch ein medizinisches System mit einer MRT-Spulenvorrichtung, einem medizinischen Instrument, das über der zumindest einen Interventionsöffnung anordenbar ist, und einem Magnetresonanzsystem, mit dem die MRT-Lokalspulenvorrichtung gekoppelt ist.

Die Erfindung offenbart auch ein Verfahren zum Umwandeln einer MRT-Lokalspulenvorrichtung in eine Diagnose-Lokalspulenvorrichtung oder eine Interventions-Lokalspulenvorrichtung und umgekehrt mit dem Schritt des Bewegens eines ersten Trägerbereichs mit Antennenelementen gegenüber einem zweiten Trägerbereich mit Antennenelementen. Befindet sich der erste Trägerbereich in der Diagnosestellung, arbeitet die MRT-Lokalspulenvorrichtung als Diagnose-Lokalspulenvorrichtung. Befindet sich der erste Trägerbereich in der Interventionsstellung, arbeitet die MRT-Lokalspulenvorrichtung als Interventions-Lokalspulenvorrichtung.

Das Verfahren kann so weitergebildet sein, wie hinsichtlich der Vorrichtung beschrieben ist.

Für bestimmte Anwendungen, z. B. die Biopsie der Prostata, ist es besonders vorteilhaft, dass die MRT-Lokalspulenvorrichtung mit zwei fensterartigen Interventionsöffnungen versehen ist. Die entsprechende MRT-Lokalspulenvorrichtung ist bevorzugt mittels eines zweiteiligen Trägers ausgebildet, wobei einer der Trägerbereiche dann die beiden Interventionsöffnungen aufweist. Dadurch kann insbesondere eine Biopsie an der rechten und der linken Seite der Prostata durchgeführt werden, ohne dass die Lokalspulenvorrichtung gewechselt oder verschoben werden muss. Jeder der Trägerbereiche ist bevorzugt zweireihig ausgebildet, wobei nach der hier bevorzugten Ausführungsform die beiden Interventionsöffnungen in derselben Reihe eines der beiden Trägerbereiche angeordnet sind. So kann insbesondere bei der Prostata-Biopsie der Befund steril, sicher und gut zugänglich punktiert werden, ohne Gefäße oder Organe, insbesondere auch den Dickdarm, zu verletzen. Hierzu wird der die Interventionsöffnungen aufweisende Trägerbereich der Lokalspulenvorrichtung so platziert, dass etwa die Pofalte auf einer Line etwa in der Mitte zwischen den Interventionsöffnungen oder etwa in der Mitte der Interventionsöffnung liegt.

Bei der zweireihigen Ausführungsform können die Antennenelemente für die gezielte Diagnostik des Beckens so angeordnet werden, dass sie in einer Reihe im mittleren Bereich des kleinen Beckens vergleichsweise dicht aneinander liegen, um eine maximale Auflösung des MRT-Signals im Bereich der Prostata und anderer sich dort befindender Organe zu erhalten. In der jeweils anderen Reihe mit der oder den Interventionsöffnungen können die Antennenelemente einen vergleichsweise größeren Abstand aufweisen, wobei jedenfalls Kanäle bzw. Antennenelemente im unmittelbaren Randbereich der wenigstens einen Interventionsöffnung vorgesehen sein sollten. Wenigstens ein Antennenelement sollte dann zwischen den Interventionsöffnungen angeordnet sein. Dieses Antennenelement liegt dann im Anwendungsfall direkt über der Prostata. Die Zahl der Antennenelemente ist zunächst beliebig, bevorzugt sind wenigstens 3 Antennenelemente, insbesondere auch 8 Antennenelement, in der jeweils zweiten Reihe und wenigstens 3 Antennenelement, insbesondere auch 5 oder 6 Antennenelement, in der jeweils ersten Reihe.

Durch die zweiteilige Ausführung der Lokalspulenvorrichtung ist es zudem möglich, den die Interventionsöffnung(en) aufweisenden Teil der Lokalspulenvorrichtung um 180° zu drehen, so dass eine maximal gute Auflösung des MRT-Signal bei der Diagnostik einerseits und bei Intervention bzw. Punktion andererseits gewährleistet ist.

Die Erfindung wird nachfolgend anhand der Figuren 1 bis 15 näher erläutert. Die Figuren zeigen nicht einschränkende Ausführungsformen der Erfindung.
Figur 1 zeigt eine perspektivische Darstellung einer erfindungsgemäßen MRT-Lokalspulenvorrichtung mit einer fensterartigen Interventionsöffnung;
Figur 2 zeigt eine Ausschnittsvergrößerung der Lokalspulenvorrichtung im Bereich der Interventionsöffnung;
Figur 3 zeigt eine perspektivische Ansicht der Lokalspulenvorrichtung im offenen Zustand;
Figur 4 zeigt eine Draufsicht eines erfindungsgemäßen Referenzelementes mit Ausschnittsvergrößerung;
Figur 5A zeigt eine erste Ausführungsform einer erfindungsgemäßen Lokalspulenvorrichtung mit Traverse und Nadelhalterung in Draufsicht in einer Interventionsöffnung;
Figur 5B zeigt eine Detailansicht der Traverse und einer Nadelhalterung aus Figur 5A;
Figur 6A zeigt eine zweite Ausführungsform einer erfindungsgemäßen Lokalspulenvorrichtung mit Traverse und Nadelhalterung in Draufsicht;
Figur 6B zeigt eine Detailansicht der Traverse und einer Nadelhalterung aus Figur 5A;
Figur 7 zeigt eine Detailansicht der Nadelhalterung;
Figur 8 zeigt eine Draufsicht auf den ersten Teil einer erfindungsgemäßen Lokalspulenvorrichtung mit einer Interventionsöffnung;
Figur 9A zeigt eine perspektivische Darstellung einer erfindungsgemäßen zweiteiligen MRT-Lokalspulenvorrichtung mit zwei fensterartigen Interventionsöffnungen;
Figur 9B zeigt eine perspektivische Darstellung einer weiteren erfindungsgemäßen zweiteiligen MRT-Lokalspulenvorrichtung mit zwei fensterartigen Interventionsöffnungen;
Figur 10A zeigt eine Draufsicht auf den ersten Teil der in Figur 9 gezeigten Lokalspulenvorrichtung mit zwei Interventionsöffnungen;
Figur 10B zeigt eine Draufsicht auf den zweiten Teil der Lokalspulenvorrichtung gemäß Figur 1 oder Figur 9;
Figur 10C zeigt Draufsichten auf weitere Ausführungsformen der ersten und zweiten Spulenteile;
Figur 11 zeigt Beispiele für unterschiedliche Formen von Antennenelementen;
Figur 12 zeigt weitere Anordnungen von Antennenelementen und Interventionsöffnungen;
Figur 13 zeigt eine praktische Anwendung der Erfindung bei der Diagnose;
Figur 14 zeigt eine praktische Anwendung der Erfindung bei der Intervention; und
Figur 15 zeigt schematisch den Einsatz der erfindungsgemäßen Lokalspulenvorrichtung bei einer Punktion der Prostata.

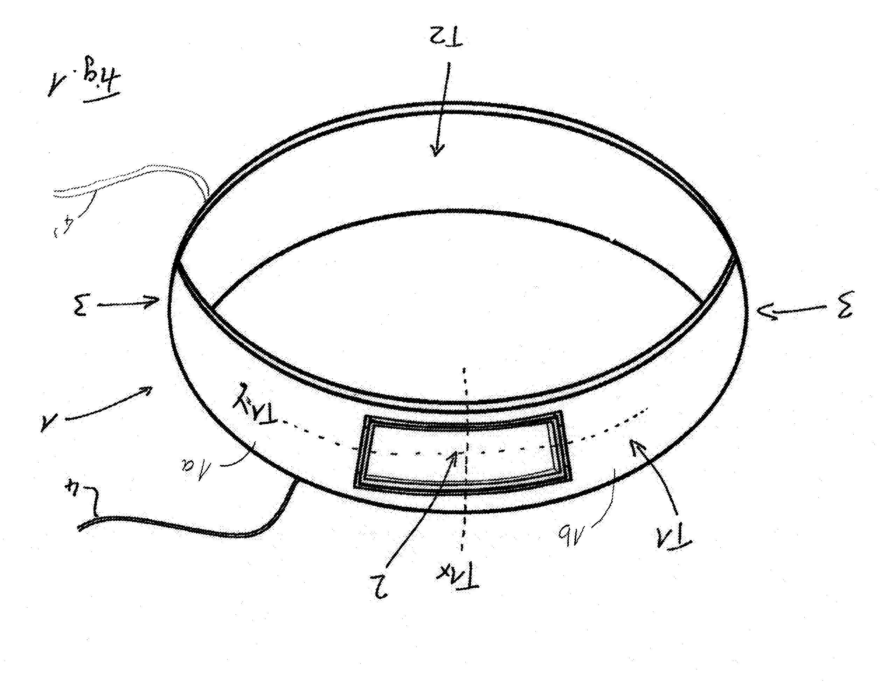

Die folgenden Ausführungen gelten sinngemäß für Spulenvorrichtungen mit einer oder mehreren Interventionsöffnung(en). Die Eigenschaften der nachfolgend, insbesondere in den Figuren 1 bis 6B, beschriebenen Interventionsöffnung können daher bei Lokalspulenvorrichtungen mit einer oder einer Mehrzahl Interventionsöffnungen vorhanden sein. Ähnliches gilt auch für die Elemente, die mit der Interventionsöffnung zusammenwirken.

Die in Figur 1 dargestellte Lokalspulenvorrichtung 1 weist wenigstens eine Interventionsöffnung 2 in einem Träger 1a, 1 b auf. Der Träger ist in Gürtel- oder Manschettenform ausgebildet. Der Träger ist bevorzugt flexibel, kann aber auch starre Abschnitte oder Bereiche aufweisen oder komplett starr ausgebildet sein können. In diesem Fall müssen zur formschlüssigen Anlage an einem zu untersuchenden Patienten Polsterteile oder dergleichen eingelegt werden, damit die Lokalspulenvorrichtung gegenüber dem Patienten während der Behandlung oder Messung nicht verschoben werden kann. Bevorzugt ist eine flexible Manschette vorgesehen, die an wenigstens einer Verbindungsstelle 3 über eine Verbindungseinrichtung zusammengeführt ist. Die Verbindung ist bevorzugt so, insbesondere seitlich, platziert, dass der zu untersuchende Patient nicht drauf liegt. Insbesondere kann die Lokalspulenvorrichtung 1 zwei voneinander trennbare Trägerbereiche T1 und T2 aufweisen, wobei die wenigstens eine Interventionsöffnung 2 in einem ersten Trägerbereich T1 vorgesehen ist. Die Signale der Lokalspulenvorrichtung 1 werden über zwei Sammelleitungen 4, 4' an eine Auswerteeinrichtung eines (nicht gezeigten) Tomographen übermittelt.

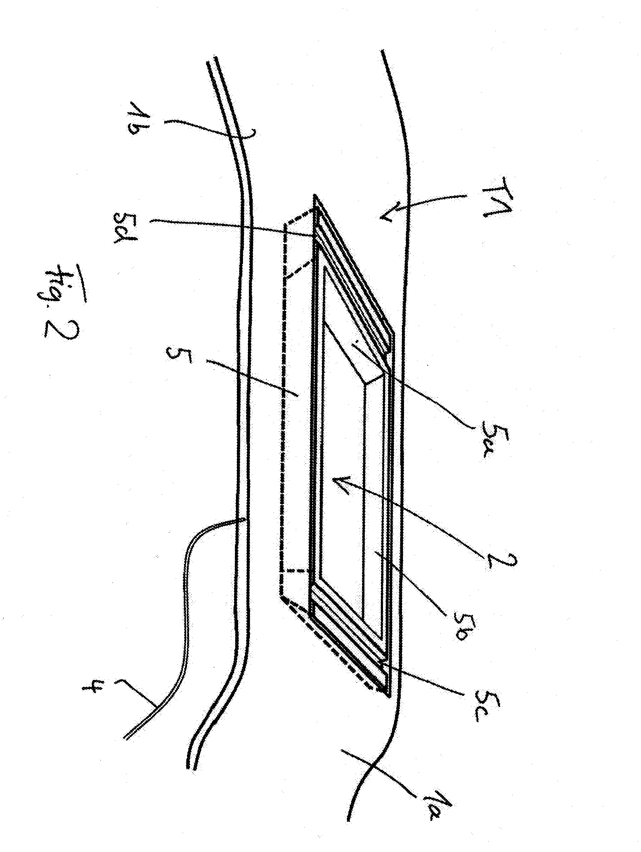

In Figur 2 ist der Bereich der wenigstens einen Interventionsöffnung 2 genauer dargestellt. Man erkennt zwischen den beiden die Manschette 1 bildenden Teilen 1a, 1 b der Lokalspulenvorrichtung einen Rahmen 5, der die Interventionsöffnung 2 umgibt. Die flexiblen Teile 1a und 1 b des Trägers, in denen die Antennenelemente K vorgesehen sind, sind mit dem im gezeigten Beispiel rechteckigen Rahmen 5 an gegenüber liegenden Seiten verbunden. Der Rahmen 5 weist an seiner Innenseite abgeschrägte Gleitflächen 5a, 5b auf, die an einander angrenzenden Seiten des Rahmens 5 vorgesehen sind und wobei die Gleitflächen zur Mitte des Rahmens 5, also zur Mitte der Interventionsöffnung 2 hin abfallen. Weiter erkennt man an gegenüber liegenden Seiten des Rahmens 5 Führungen 5c, 5d, an denen Anbauteile angebracht bzw. geführt werden können. Im gezeigten Beispiel liegen die Führungen 5c, 5d zwischen der Interventionsöffnung 2 und jeweils einem Manschettenteil 1a, 1 b der Lokalspulenvorrichtung 1.

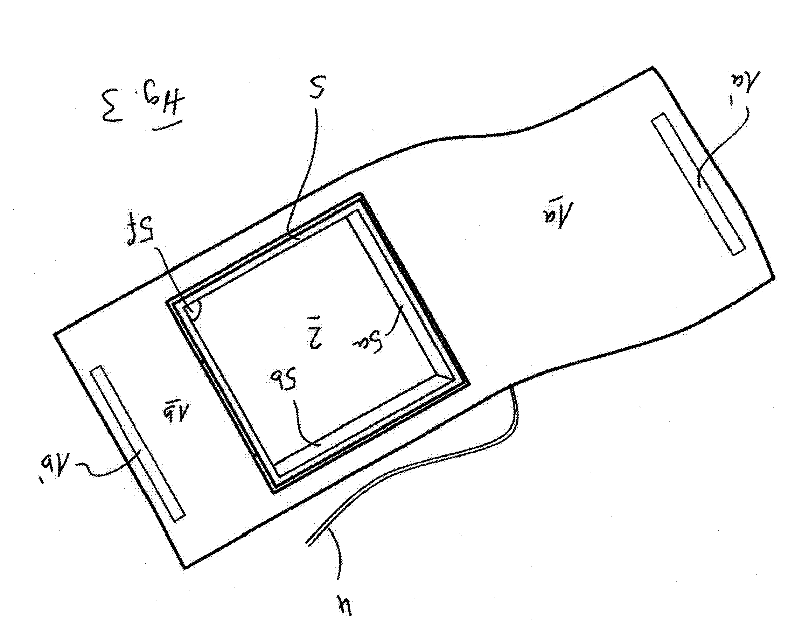

Figur 3 zeigt nochmals die Lokalspulenvorrichtung 1 in ihrer offener Form, wobei zum Schließen der Manschette in diesem Beispiel Klettverbinder 1a' und 1b' an vom Rahmen 5 entfernt liegenden jeweiligen Enden der beiden Manschettenteile 1a, 1 b vorgesehen sind. Die Gleitflächen 5a, 5b dienen zur definierten Ausrichtung eines Referenzelements, welches für die genaue Positionierung und Ortung von Koordinatenpunkten im MRT-Bild sorgt. Dadurch können im Bild gemessene Daten einer relativen Lage zum Rahmen 5 eindeutig zugeordnet werden. Dazu ist es erforderlich, dass das im MRT-Bild sichtbare Referenzelement während der Bildaufnahme nicht verschoben werden kann.

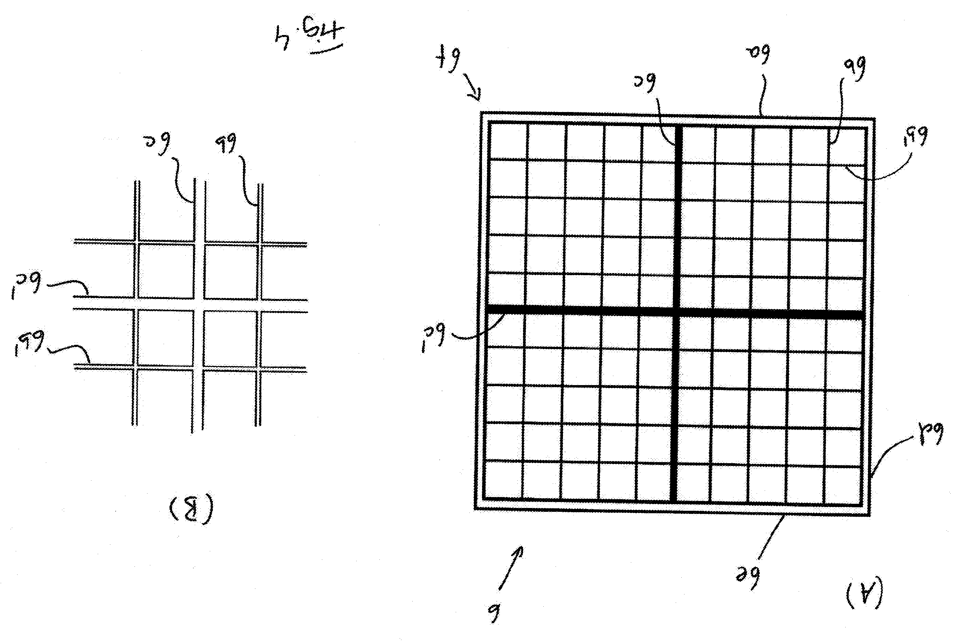

Das Referenzelement 6, welches in der Figur 4 gezeigt ist, ist bevorzugt ein Gitter, dessen Umrissform an die Form der Interventionsöffnung 2 angepasst ist, so dass es in die Interventionsöffnung 2 eingelegt werden kann. Das Referenzelement wird bei der Diagnose oder Intervention der Prostata am Rücken des Patienten angeordnet. Das Gitter ist hier ein Röhrengitter. Im Innern der Röhren 6b, 6b', 6c, 6c' befindet sich ein im MRT-Bild sichtbares Mittel, beispielsweise Fischöl. Ein Röhrenkreuz, bevorzugt das mittlere im hier gezeigten Rechteckgitter, weist dickere Röhren 6c, 6c' auf, so dass es im MRT-Bild von den übrigen Röhren eindeutig zu unterscheiden ist und im MRT-Bild eine genaue Lage des Befundes relativ zum Gitter erlaubt. Wird das Referenzelement 6 in den Rahmen eingelegt, so liegen die Seiten 6d, 6e auf den Gleitflächen 5a, 5b des Rahmens 5 auf. Durch die Schwerkraft "rutscht" das Gitter 6 in diesem Bereich an den Gleitflächen 5a, 5b nach unten, so dass die Ecke 6f des Gitters 6 in die Ecke 5f des Rahmens gedrückt wird und sicher dort anliegt. Zweckmäßigerweise befindet sich wenigstens im Bereich der Ecke 5f eine Auflage, so dass das Gitter 6 nicht aus dem Rahmen 5 nach unten herausfallen kann.

Mit dem Referenzelement 6 können im MRT-Bild die relative Lage zwischen Lokalspulenvorrichtung 1 und zu beobachtendem Befund ermittelt werden. Insbesondere lässt sich so auch die Einstichstelle einer Punktionsnadel und/oder der Eingangsweg für ein Instrument bestimmen und am Körper des Patienten markieren. Zur Punktion wird das Referenzelement dann entfernt und es steht zur weiteren Behandlung wieder die Interventionsöffnung 2 zur Punktion und/oder Intervention vollflächig zur Verfügung.

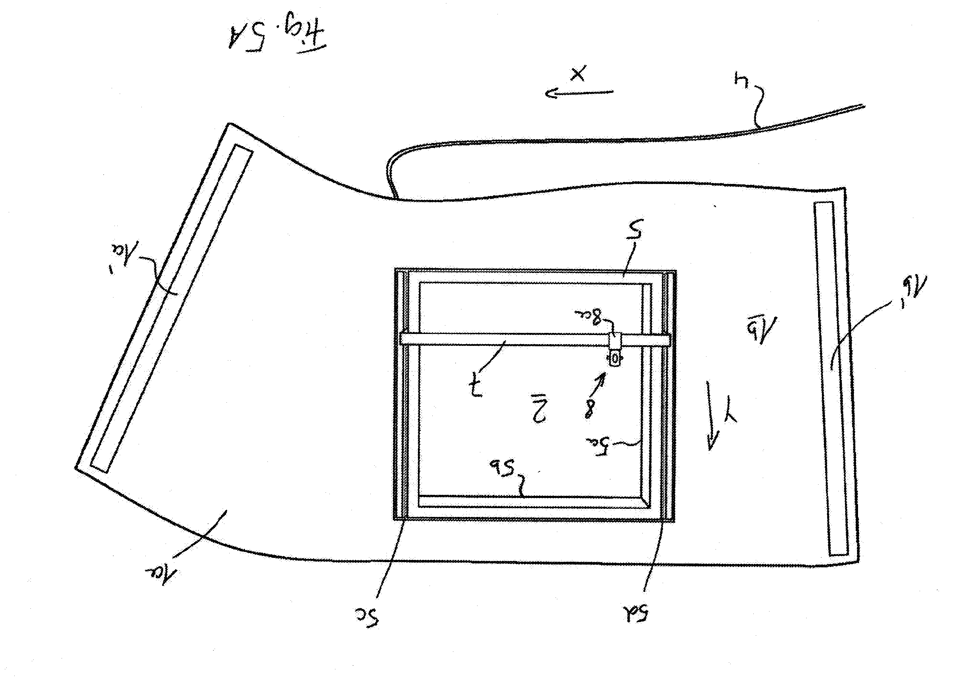

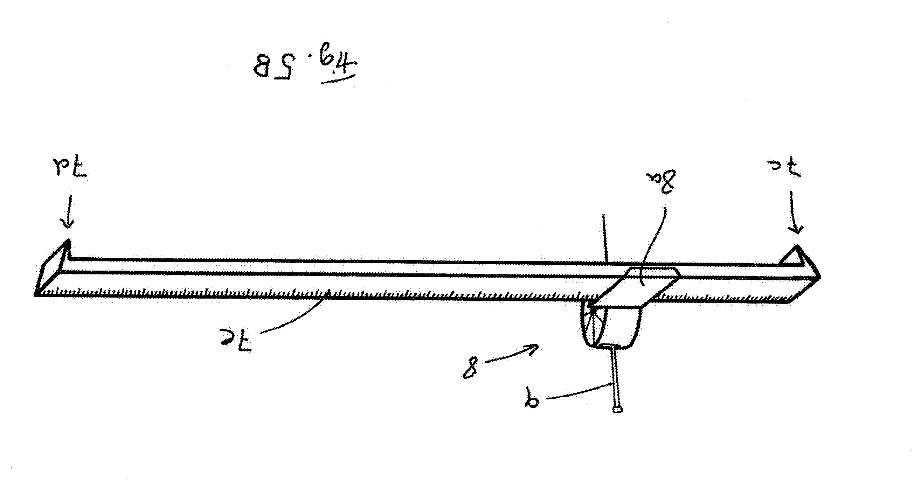

In Figur 5A ist eine Draufsicht auf eine erfindungsgemäße Lokalspulenvorrichtung 1 gezeigt, bei der die Interventionsöffnung 2 bzw. der Rahmen 5 mit einem oder mehreren Anbauteilen 7, 8 bestückt ist. Idealerweise weist die Interventionsöffnung für die meisten Anwendungen eine Erstreckung von etwa 7 cm bis etwa 20 cm (vorzugsweise etwa 8 cm bis etwa 10 cm) in Trägerlängsrichtung bzw. Manschettenlängsrichtung X und eine Erstreckung von etwa 7 cm bis etwa 20 cm (vorzugsweise etwa 8 cm bis etwa 10 cm) in der Richtung Y quer zum Träger bzw. zur Manschette 1 auf. Eines der gezeigten Anbauteile 7 ist hier als Traverse vorgesehen, welche die Interventionsöffnung 2 in Richtung X überspannt und in Führungen 5c, 5d geführt ist. Die Traverse 7, die sich in Richtung X erstreckt, ist in den bevorzugt als Nuten 5c, 5d ausgebildeten Führungen in Y-Richtung verschiebbar gelagert und kann durch geeignete Fixierelemente (Schrauben oder dergleichen) in jeder beliebigen Position fixiert werden.

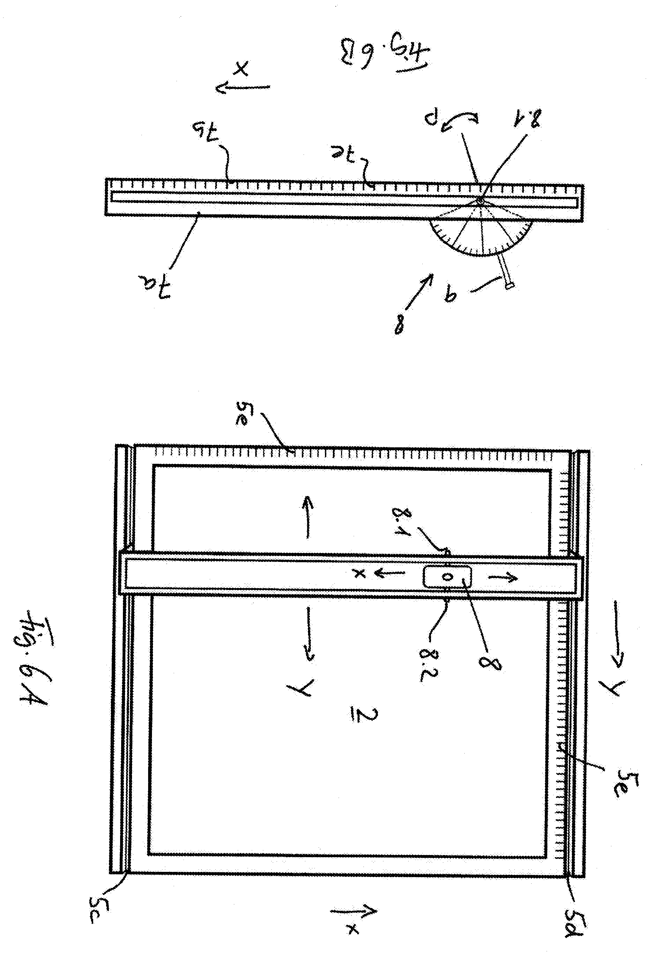

Die Traverse 7, die in der Figur 6 näher gezeigt ist, ist mit ihren Enden 7c und 7d in oder an den Führungen 5c, 5d geführt. Bevorzugt sind die Traverse 7 und die Führungen 5c, 5d mit Hinterschneidungen ausgebildet, so dass sich die Traverse 7 beim Gebrauch nicht aus der Führung löst bzw. vom Rahmen getrennt wird. Der Rahmen 5 und die Seiten desselben sowie die Traverse 7 haben im gezeigten Beispiel Skalenstriche oder sonstige Markierungen 5e (in metrischen oder sonstigen Einheiten), um die relative Position von der Traverse 7 zum Rahmen 5 und auch die Position des unten beschriebenen Instrumentenhalters bzw. Nadelhalters 8 zur Traverse 7 genau einstellen zu können. An der Traverse 7, die im mittleren Bereich gegenüber den Seiten 7d, 7c abgesenkt sein kann, aber nicht abgesenkt sein muss, ist ein Instrumentenhalter bzw. Nadelhalter 8 zur Aufnahme und Halterung eines Instrumentes bzw. einer Nadel 9 vorgesehen. Der Instrumentenhalter bzw. Nadelhalter 8 ist an der Traverse 7 bzw. jedenfalls relativ zum Rahmen 5 in wenigstens einer Richtung X verschiebbar ausgebildet. Im gezeigten Beispiel ist der Instrumentenhalter bzw. Nadelhalter 8 an einem Schlitten 8a angebracht und darüber gegenüber der Traverse 7 verschiebbar. Die Nadel 9 kann in unterschiedlichen Winkelstellungen eingestellt und Mittels der Traverse 7 zur Einstichstelle geführt werden.

In den Figuren 6A und 6B ist eine alternative Ausführungsform der Traverse 7 gezeigt. Der Rahmen ist derselbe wie bei der vorangegangenen Ausführungsform, wobei hier zusätzlich Skalen oder Markierungen 5e am Rahmen vorgesehen sind (die auch bei den anderen Ausführungsformen vorhanden sein können), um insbesondere die Lage der Gitterlinien 6b, 6c des Referenzelements 6 relativ zum Rahmen 5 bestimmen zu können. Die Markierungen 5e, die an einer oder mehreren Seiten des Rahmens 5 vorgesehen sein können, dienen somit also als Lineal zur Bestimmung von Positionen auf dem Patientenkörper, etwa zur Bestimmung der Einstichstelle bei der Biopsie. Die Nadelhalterung ist hier in der Traverse 7 geführt und lässt sich so in Richtung X, also in Längsrichtung der Traverse 7 verschieben. Mittels Führungselementen 8.1, 8.2 ist die Instrumentenhalterung bzw. Nadelhalterung 8 zwischen übereinander angeordneten Führungsschienen 7a, 7b der Traverse geführt. Wie man in Figur 6B erkennt, kann die Instrumentenhalterung bzw. Nadelhalterung 8 so auch in der Führung 7a, 7b in einer Ebene senkrecht zur Verschieberichtung Y der Traverse 7 in Richtung des Pfeils P geschwenkt werden. Eine Skala 10 dient der Ablesung und Einstellung des gewünschten Nadelwinkels.

An dem Instrumentenhalter kann ein beliebiges medizinisches Instrument angeordnet sein. Das medizinische Instrument kann ein Diagnoseinstrument oder ein Operationsinstrument sein. Im Folgenden wird die Erfindung im Kontext eines Nadelhalters detaillierter beschrieben.

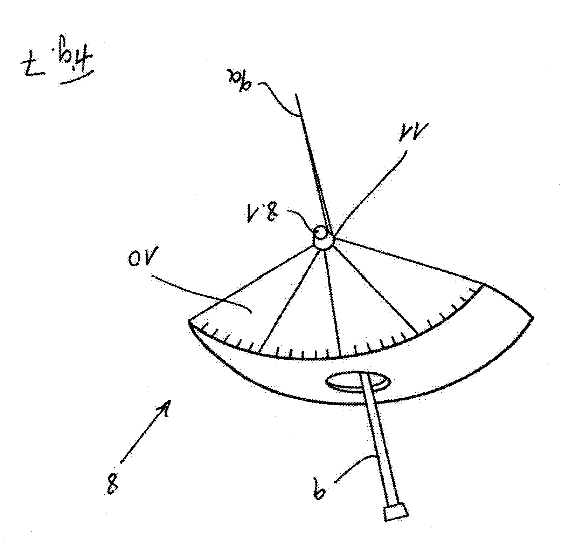

Die schematische Figur 7 zeigt den Nadelhalter 8 detaillierter. Die Nadel 9 ist in der Halterung 8 eingespannt und kann im gezeigten Beispiel um das Drehlager 11 oder dergleichen im Winkel verstellt werden. Das in den Figuren 6A, 6B gezeigte Führungselement 8.1 bildet so auch die Drehachse. Über eine Skala 10 auf der Halterung 8 oder/und dem Führungselement 8.1 oder Strichmarkierungen kann der Winkel je nach Vorgabe auf den vorgegebenen Einstichwinkel eingestellt werden. Zudem ist die Nadel in der Halterung 8 verschiebbar und kann durch einen nicht näher gezeigten Fixiermechanismus in jeder Verschiebeposition fixiert werden, um die Nadel 9 mit ihrer Spitze 9a an die Einstichposition heranfahren zu können. Bevorzugt ist die Nadel 9 dabei so geführt, dass Sie durch die Drehachse der Nadelhalterung 8 verläuft.

Die Positionen der Anbauteile können nach einer Weiterbildung der Erfindung auch elektronisch erfasst und ggf. auch elektrisch gesteuert werden, so dass eine Computer unterstütze Positionierung der Nadel 9 an der Einstichstelle prinzipiell möglich ist.

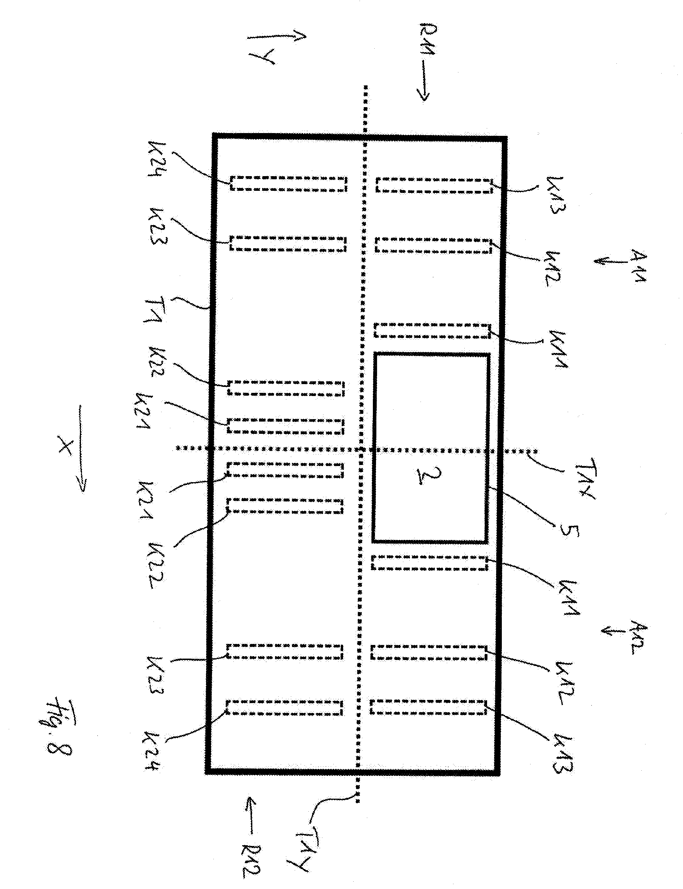

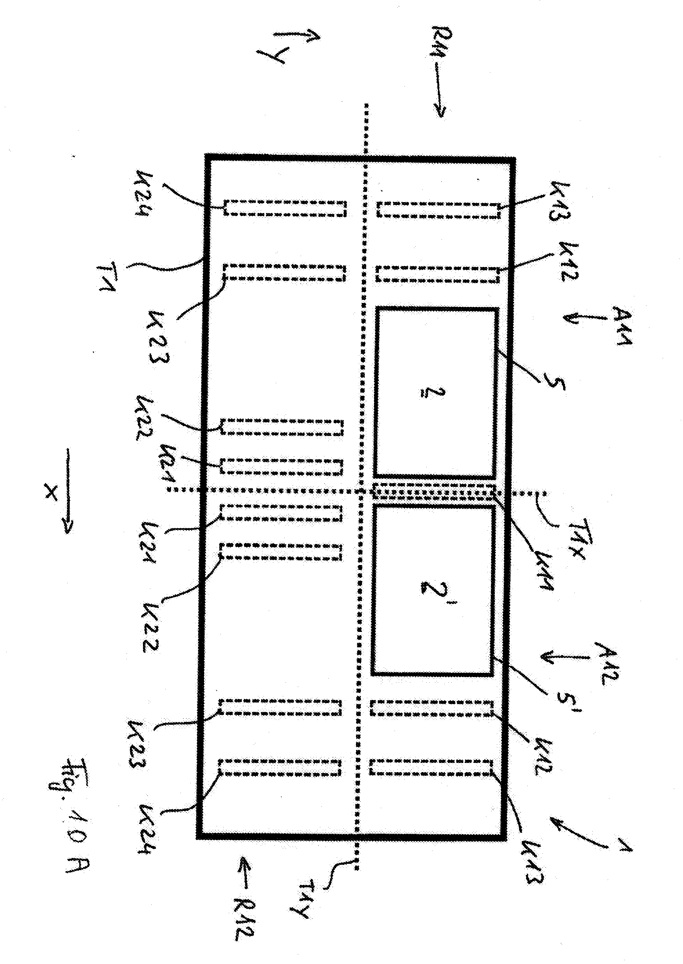

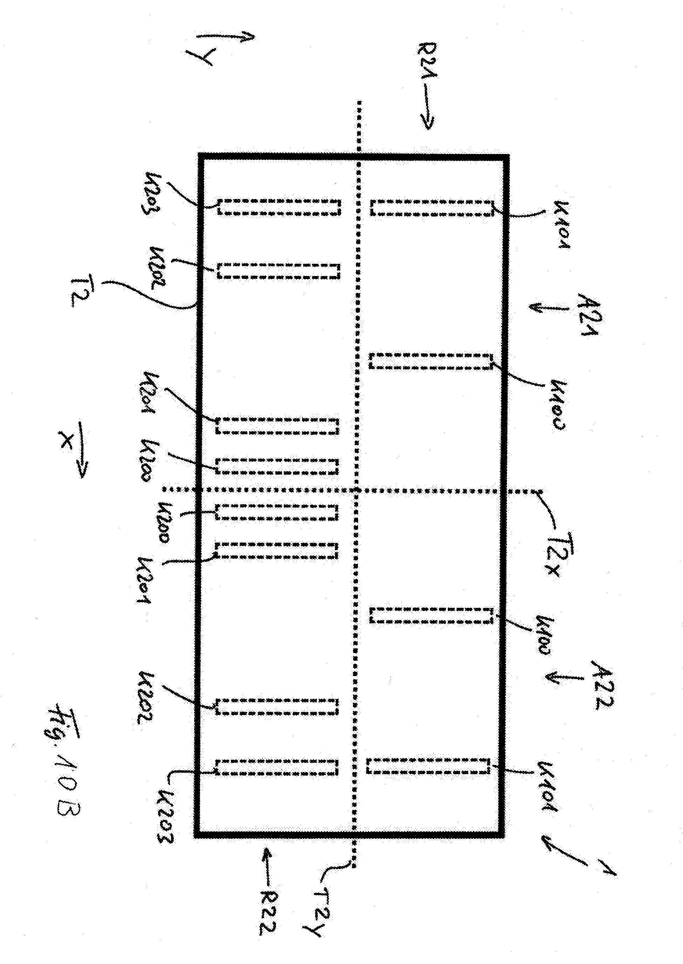

In Figur 8 ist eine schematische Ansicht des ersten Trägerbereichs T1 einer erfindungsgemäßen Lokalspulenvorrichtung mit einer Interventionsöffnung 2 dargestellt. Es handelt sich um den oberen Teil der Lokalspulenvorrichtung 1, also dem Teil der zweiteiligen Lokalspulenvorrichtung 1, welcher auf dem Patienten positioniert wird. Der "untere" zweite Teil T2 der Lokalspulenvorrichtung wird nachfolgend noch anhand des in den Figuren 10A und 10B gezeigten Ausführungsbeispiels näher beschrieben. Die dortigen Ausführungen gelten auch für das in Figur 8 gezeigte Ausführungsbeispiel, der Unterschied ist lediglich, dass das vorliegende Beispiel lediglich eine Interventionsöffnung 2 aufweist. Ebenso lässt sich diese Beschreibung auch auf die Figuren 10A, 10B lesen.

Die Interventionsöffnung ist bevorzugt in der in der Zeichnung oberen Reihe R11 mittig vorgesehen, d. h., die Interventionsöffnung ist bevorzugt spiegelsymmetrisch zur gedachten Mittellinie T1X angeordnet, welche den ersten Teil in die Abschnitte A11 und A12 unterteilt. Diese können gleich groß sein, dies ist jedoch nicht zwingend erforderlich. Eine horizontale gedachte Linie T1Y teilt den ersten Trägerbereich T1 in zwei Reihen R11 und R12 auf. In den Reihen R11 und R12 sind Antennenelemente bzw. Kanäle K11-K13 in der oberen Reihe R11 und K21-K24 in der unteren Reihe angeordnet. Bevorzugt liegen entsprechende Antennenelemente oder Kanäle K11 in unmittelbarer Nähe des Randes oder des Rahmens 5 der Interventionsöffnung 2, um dort eine ausreichende Signalstärke zu erhalten. In der oberen Reihe sind mindestens 3 Antennenelemente vorgesehen, wobei es möglich ist, ein Antennenelement in der Mitte des Fensters (nicht gezeigt) etwa auf der Linie T1X anzuordnen. Die übrigen nicht zur Interventionsöffnung 2 benachbarten Kanäle sind bevorzugt äquidistant auf die gesamte Längserstreckung des Teils 1 verteilt. Generell empfiehlt es sich, die Dichte der Kanäle bzw. Antennenelemente dort zu erhöhen, wo im Anwendungsfall die genausten MRT-Signale benötigt werden, d. h. an den Stellen der Spulenvorrichtung, die dem zu untersuchenden Körperteil am nächsten liegen. So sind in der unteren Reihe im Bereich der Mittellinie im gezeigten Beispiel vier Antennenelemente bzw. Kanäle K21, K22 dicht, bevorzugt so dicht wie technisch machbar und sinnvoll angeordnet, die übrigen Antennenelemente bzw. Kanäle K23, K24 weisen größere Abstände auf, da insbesondere im Bereich des Bauchs an diesen Stellen keine so große Signalintensität bzw. Bildauflösung benötigt wird. Der Rand des großen Beckens wird von den Antennenelementen K12, K13, K23, K24 erfasst.

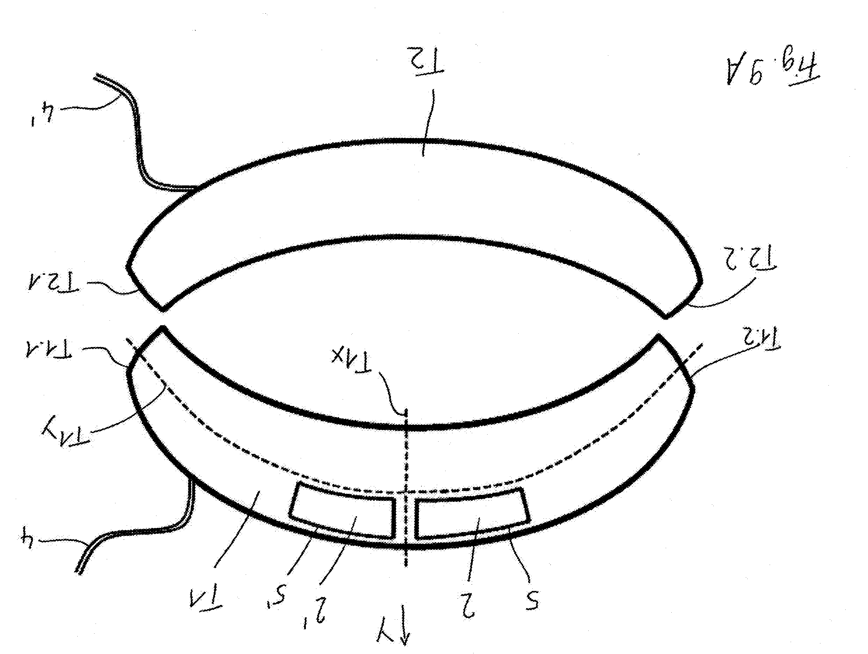

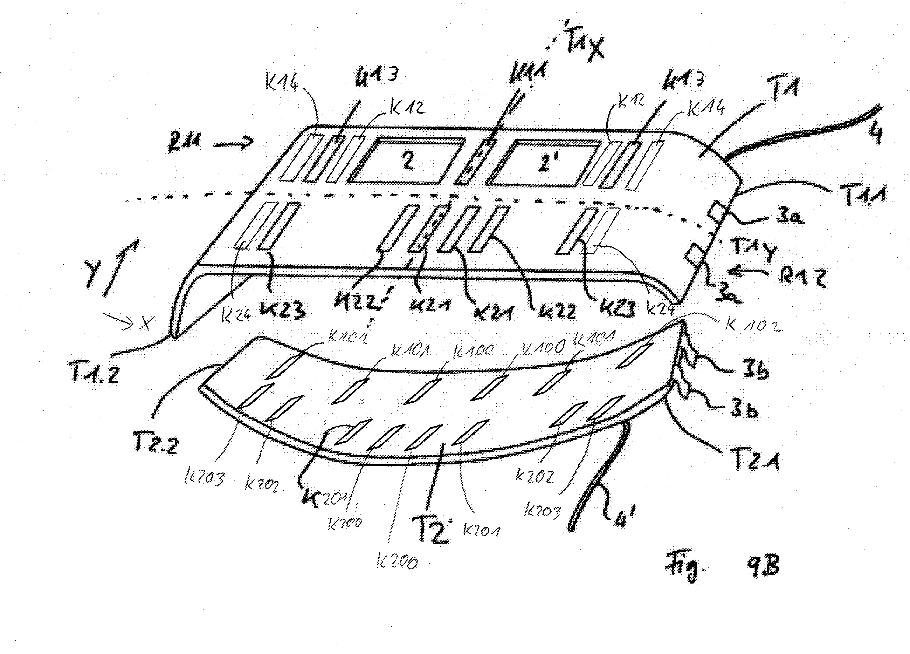

Eine besondere Ausführungsform der Erfindung ist in den Figuren 9A, 9B, 10A und 10B schematisch dargestellt. Die Lokalspulenvorrichtung 1 ist in dieser Ausführungsform insbesondere für die Diagnostik und Biopsie der Prostata geeignet und weist zwei Trägerbereiche, d.h. einen oberen ersten Trägerbereich T1 und einen unteren zweiten Trägerbereich T2, auf, welche an den jeweiligen Enden (in X-Richtung) miteinander verbunden werden. "Oben" und "unten" bezieht sich hier auf die bevorzugte Verwendung, bei der der auf dem Bauch liegende Patient mit seinem Becken auf dem unteren zweiten Trägerbereich T2 liegt und der obere erste Trägerbereich seinerseits auf dem Patient aufliegt. Im Folgenden wird nur von erstem und zweitem Trägerbereich gesprochen. Die beiden Trägerbereiche weisen Enden T1.1, T1.2 bzw. T2.1, T2.2 auf, an denen sie miteinander etwa über Klettverschlüsse, Gurte oder ähnliches miteinander verbunden werden können, um die Lokalspulenvorrichtung 1 insgesamt am Körper des Patentien möglichst nicht verrutschbar zu befestigen. Die beiden Teile lassen sich getrennt über Signalleitungen 4, 4' ansteuern bzw. auslesen.

Die in Figur 9B gezeigte zweiteilige Lokalspulenvorrichtung entspricht im Wesentlichen der aus Figur 9A. Die Lokalspulenvorrichtung und der erste Trägerbereich T1 befinden sich in ihrer Diagnosestellung. Der Unterschied ist hier, dass der obere Trägerbereich T1 einen im Wesentlichen planen und wenigstens relativ starren Mittelteil aufweist und an den Enden T1.1, T1.2 flexibel oder in Richtung des zweiten Trägerbereich gebogen ausgebildet ist. Dadurch wird das Arbeiten in diesem Bereich erleichtert und insbesondere können durch die plane Ausbildung wenigstens im Bereich der Fenster oder Interventionsöffnungen 2, 2' das Aufsetzen der Traversen oder von Referenzelementen und deren Handhabung erleichtert werden. Die Enden können wie hier an einer Seite gezeigt beispielsweise über Klettverschlüsse 3a, 3b oder Gurte miteinander verbunden werden, wobei der Abstand zwischen den Trägerbereichen T1, T2 je nach Größe des Patienten so variiert werden kann. Der zweite Trägerbereich T2 ist bevorzugt flexibel ausgebildet, so dass er beim darauf liegenden Patienten keine Druckstellen hinterlässt.

Bei einer Untersuchung der Prostata liegt der Patient mit dem Rücken auf dem zweiten Trägerbereich T2. Bei einer Intervention der Prostata hingegen liegt der Patient mit dem Bauch auf dem zweiten Trägerbereich T2.

Eine Mehrzahl von Antennenelementen K200, 201 ist sowohl bei der Diagnose als auch bei der Intervention im Bereich des kleinen Beckens angeordnet. Diese Antennenelemente K200, 201 befinden sich in Längsrichtung X des zweiten Trägerbereichs T2 mittig und in Querrichtung Y des zweiten Trägerbereichs T2 unten. In dem in Figur 9B dargestellten Beispiel befinden sich vier Antennenelemente K200, 201 im Bereich des kleinen Beckens, beispielsweise in sagittaler (dorsaler) Richtung unter dem kleinen Becken. Dadurch kann eine gute Bildgebung der Prostata erzielt werden. In Querrichtung Y des zweiten Trägerbereichs T2 oben befindet sich eine Mehrzahl von Antennenelementen K100, K101, K102 im Wesentlichen äquidistant entlang der Längsrichtung X über den zweiten Trägerbereich T2 verteilt. Bei dem in Figur 9B gezeigten Beispiel befinden sich sechs Antennenelemente K100, K101, K102 entlang der Längsrichtung X des zweiten Trägerbereichs verteilt in einem Bereich, der sich in Querrichtung Y des zweiten Trägerbereichs T2 oben befindet. Es ist jedoch möglich, die Antenennelemente K102, K102 am oberen Rand des großen Beckens in einer größeren Dichte anzuordnen. Am unteren Rand des großen Beckens befinden sich je zwei Antennenelemente K202, K203. Diese Antennenelemente K202, K203 befinden sich in Längsrichtung des zweiten Trägerbereichs T2 jeweils am Rand und in Querrichtung des zweiten Trägerbereichs T2 unten.

Der in Figur 10A gezeigte erste Trägerbereich T1 weist zwei Interventionsöffnungen 2, 2' auf, die von Rahmen 5, 5' eingefasst sind. Die Ausgestaltung der Interventionsöffnungen 2, 2' bzw. Rahmen 5, 5' ist genauso möglich, wie oben bei den anderen Ausführungsformen beschrieben, so dass auf die dortige Beschreibung zur Vermeidung von Wiederholungen verwiesen wird. Die Anordnung der Interventionsöffnungen 2, 2' kann wie hier gezeigt in Richtung X nebeneinander beidseits einer ersten Teilungslinie T1X, die den ersten Teil in zwei Abschnitte A11, A12 unterteilt, und oberhalb einer zweiten Teilungslinie T1Y erfolgen, welche den ersten Trägerbereich T1 in zwei Reihen R11 und R12 unterteilt. Im gezeigten Beispiel liegen die Interventionsöffnungen 2, 2' also in der ersten Reihe R11, jeweils eine Interventionsöffnung 2 liegt im Abschnitt A11 und eine Interventionsöffnung 2' liegt im Abschnitt A12. Denkbar ist auch eine Ausführungsform, bei der beide Interventionsöffnungen jeweils in einer Reihe, aber untereinander in demselben Abschnitt liegen.

Weiter erkennt man mit den Bezugszeichen K11...K24 Sende- und/oder Empfangseinrichtungen, die als Antennenelemente bezeichnet werden. Die Antennenelemente werden in der medizinischen Praxis auch als Kanäle bezeichnet. Die Lokalspulenvorrichtung wird in der medizinischen Praxis auch als MRT-Spule oder lediglich Spule bezeichnet. Die Anzahl der Antennenelemente bzw. Kanäle pro Fläche (und damit letztlich die Auflösung des MRT-Signals) ist hier offen gelassen und kann variiert werden. Es können insbesondere die Signale mehrerer Sende- und/oder Empfangseinrichtungen in der Lokalspulenvorrichtung 1 zu einem Kanal zusammengefasst werden. Die hier angedeutete Anordnung der Kanäle soll lediglich verdeutlichen, in welchen Bereichen der Lokalspulenvorrichtung eine höhere Kanaldichte erforderlich ist, um insbesondere bei im hier favorisierten Bereich des menschlichen Beckens gute Signale zu erhalten. Da erfindungsgemäß zwei Interventionsöffnungen 2, 2' vorgesehen sind, ist zum Erhalten eines qualitativ guten Signals wesentlich, wenigstens ein Antennenelement bzw. einen Kanal K11 zwischen den Interventionsöffnungen 2, 2' anzuordnen. Bei der Beckenuntersuchung liegt dieses Antennenelement K11 direkt über der Pofalte und damit auch direkt über der Prostata bzw. in longitudinaler Richtung des Patienten auf der gleichen Höhe wie die Prostata, so dass in diesem Bereich ausreichend gute Signalstärken realisiert werden. Das Antennenelement K11 liegt in der sagittalen (ventralen) Richtung über der Prostata. Weiter außen liegen dann noch die Kanäle K12, K13, in größerem Abstand. Die Interventionsöffnungen 2, 2' können zwei separate Interventionsöffnungen sein oder aber aus einer Interventionsöffnung gebildet werden.

In der zweiten Reihe R12, in der keine Interventionsöffnungen vorhanden sind, liegen im Bereich um die Mittellinie T1 X herum die Antennenelemente K21, K22 in geringem Abstand (so gering wie technisch machbar ist) nebeneinander, während die weiter außen liegenden Antennenelemente K23, K24 im Vergleich dazu größere Abstände zueinander aufweisen.

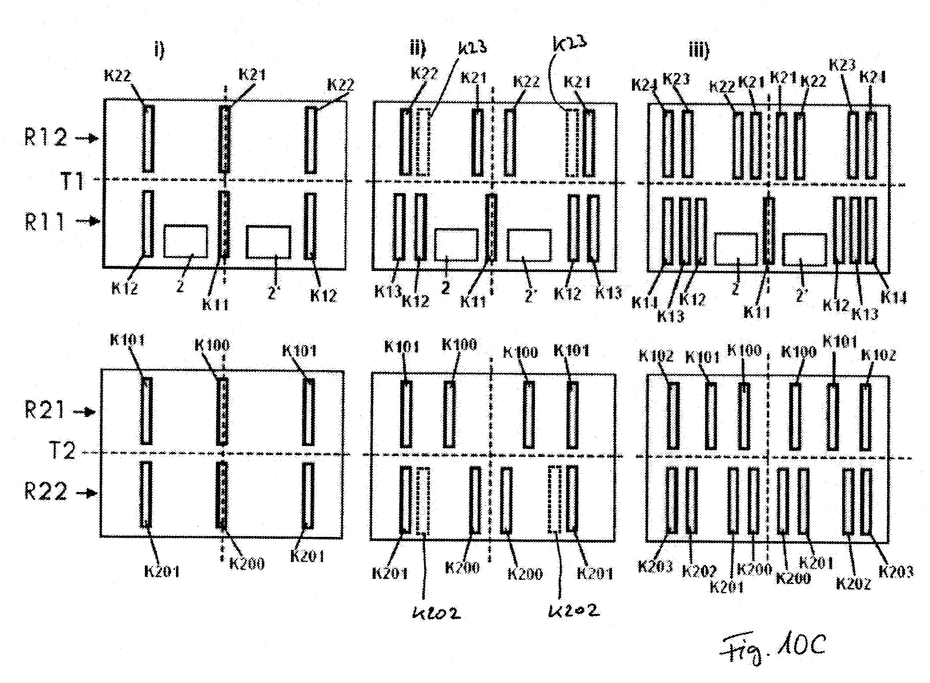

Die Ausgestaltung der des ersten Trägerbereichs wird nachstehen unter Bezugnahme auf Figur 10C iii) beschrieben, wobei zu beachten ist, dass sich der erste Trägerbereich T1 in Figur 9B in seiner Diagnosestellung und in Figur 10c iii) in der Interventionsstellung befindet.

Der in Figur 10B gezeigte zweite Trägerbereich T2 der Lokalspulenvorrichtung 1 ist durch eine Teilungslinie T2X in die Abschnitte A21 und A22 und durch eine Teilungslinie T2Y in Reihen R21 und R22 unterteilt. Die zweite Reihe R22 des zweiten Trägerbereich weist bevorzugt dieselbe Kanalanordnung der Antennenelemente K200 bis K203 auf wie die zweite Reihe R12 des ersten Trägerbereichs T1 (siehe Figur 10A). In der ersten Reihe, die bei der Beckendiagnostik Signale im Bereich des großen Beckens liefern soll, weist lediglich vier relativ weit voneinander beabstandete Antennenelemente K100, K101 auf. Es können mehr Antennenelemente, insbesondere 8 Antennenelemente, angeordnet werden, die bevorzugt gleich beabstandet sein können, wenn dies beispielsweise bei der Beckenuntersuchung gewünscht ist. Diese sind aber aufgrund der Beckenanatomie üblicherweise nicht zwingend erforderlich. Die zweite Reihe R22 benötigt in der Mitte des Messfeldes eine höhere Auflösung.

Für die Diagnostik des Beckens liegen die Reihen R11 des ersten Trägerbereichs und R21 des zweiten Trägerbereichs übereinander. Für die MRT-gestützte Biopsie der Prostata kann dann der erste Trägerbereich um 180° gedreht werden, so dass Reihe R11 des ersten Trägerbereich dann über der Reihe R22 des zweiten Trägerbereichs T2 und die Reihe R12 des ersten Trägerbereichs T1 über Reihe R21 des zweiten Trägerbereich T2 liegt. So ist es auch bei der Biopsie möglich, im Bereich der Interventionsöffnungen 2, 2' noch ausreichend gute MRT-Signale zu erhalten, weil die Signale durch das Antennenelement K11 (über der Prostata) und die Antennenelemente K200, K201 (unter der Prostata) genutzt werden können.

In Figur 10C sind noch weitere Bespiele für die Konfiguration der Antennenelemente in der Lokalspulenvorrichtung gezeigt, wobei der erste Trägerbereich T1 jeweils oben und der zugehörige zweite Trägerbereich T2 jeweils darunter abgebildet ist.

Die ersten Trägerbereiche sind jeweils in "Biopsiestellung" angeordnet, dass heißt mit um 180° gedrehtem ersten Teil, so dass die Interventionsöffnungen in der Zeichnung unten liegen, was der Lage über der Pofalte des Patienten entspricht. Zur Diagnose werden die ersten Teile T1 wiederum um 180° gedreht, wie dies z. B. in Figur 10A der Fall ist.

Die übrige Nomenklatur entspricht der vorherigen Beschreibung.

Die unter i), ii) und iii) gezeigten Aufführungsformen der mehrteiligen Spulenvorrichtungen unterscheiden sich lediglich in der Anordnung der Antennenelemente voneinander.

Die Ausführungsform i) weist in allen Reihen jeweils drei Antennenelemente auf, wobei die Antennenelemente K11, K21, K100 und K200 hier mittig angeordnet sind und die übrigen Antennenelemente beidseits der mittig angeordneten Antennenelemente vorgesehen sind.

Bei der Ausführungsform ii) weist der erste Trägerbereich T1 in der zweiten Reihe R12 vier Antennenelemente auf, wobei die weiter zur Mitte hin liegenden Antennenelemente dicht angeordnet sind, um im Bereich des kleinen Beckens ein qualitativ gutes Signal zu erhalten. Bei einer Frau befinden sich im kleinen Becken die Gebärmutter, die Eierstöcke und die Harnblase. Bei einem Mann befinden sich im kleinen Becken die Samenblase, die Prostata und die Harnblase. In der ersten Reihe R11 ist die Antennenelementverteilung bzw. Kanalverteilung so, wie in Figur 10A gezeigt. Beim zweiten Trägerbereich T2 sind in der ersten Reihe die Antennenelemente K100, K101 bevorzugt nahezu äquidistant und symmetrisch zur Mittellinie verteilt. In der zweiten Reihe R22 entspricht die Verteilung der zweiten Reihe R12 des ersten Trägerbereichs T1. In einer Variante können zusätzlich die Kanäle K23 im ersten Trägerbereich T1 in der Reihe R12 und K202 im zweiten Trägerbereich T2 in der Reihe R22 vorgesehen sein.

In der Ausführungsform iii) entspricht der erste Trägerbereich T1 hinsichtlich der Kanalanordnung bzw. Antennenelementanordnung weitgehend der Ausführungsform in Figur 10A, wobei an den Seiten in der ersten Reihe R11 außen lediglich jeweils ein Antennenelement K14 mehr vorgesehen ist als in der Ausführungsform von Figur 10A. Der untere Trägerbereich T2 entspricht im Wesentlichen der Ausführungsform aus Figur 10B, mit dem Unterschied, dass in der ersten Reihe R21 sechs Antennenelemente anstelle von vier Antennenelementen vorgesehen sind.

Die Ausführungsform iii) entspricht der in Figur 9B gezeigten Ausführungsform, wobei sich der erste Trägerbereich T1 in Figur 9B in seiner Diagnosestellung und in Figur 10C iii) in der Interventionsstellung befindet. Ein Antennenelement K11 ist im Wesentlichen mittig zwischen den Interventionsöffnungen 2, 2' angeordnet. An den lateralen Seiten der Interventionsöffnungen, die jeweils der anderen Interventionsöffnung entgegengesetzt sind, befinden sich jeweils drei Antennenelemente K12, K13, K14. In Querrichtung des ersten Trägers T1 oben befinden sich vier Antennenelemente K21, K22, die in Längsrichtung des ersten Trägers T1 im Wesentlichen mittig angeordnet sind. Es befinden sich in Längsrichtung des ersten Trägers außen und in Querrichtung des ersten Trägers oben je zwei Antennenelemente K23, K24.

Die Antennenelemente der in den Figuren 8 bis 10 gezeigten Ausführungsbeispiele sind im Wesentlichen schmal ausgebildet. Mit anderen Worten, die Breite der Antennenelemente ist kleiner als etwa 50 %, vorzugsweise kleiner als etwa 33 %, mehr bevorzugt kleiner als etwa 25 %, höchst vorzugsweise kleiner als etwa 12,5 % der Länge des Antennenelementes. Dadurch ist es möglich, eine Mehrzahl von Antennenelementen in einem Bereich von besonderem Interesse anzuordnen.

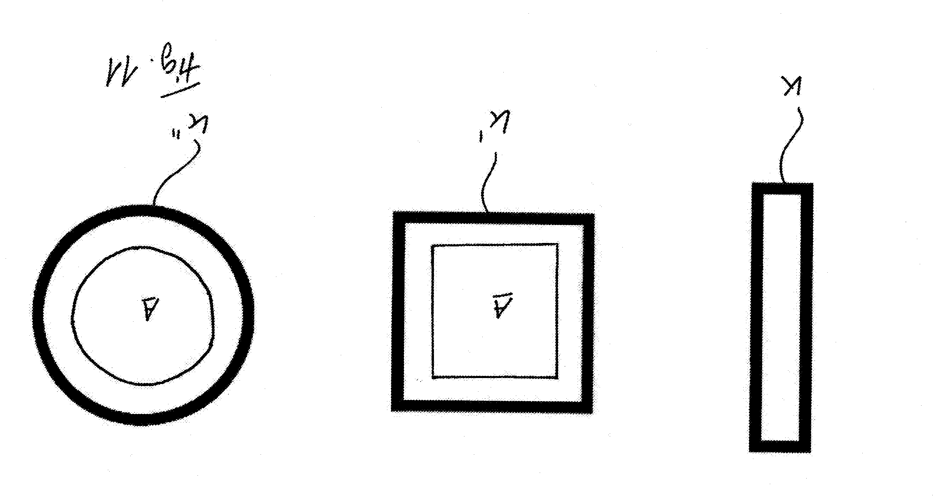

An dieser Stelle sei darauf hingewiesen, dass sie Antennenelemente oder Kanäle nicht die in den vorigen Zeichnungen dargestellte Form haben müssen, es können alle sich anbietenden Geometrien zum Einsatz kommen. In Figur 11 sind beispielhafte Antennenelemente bzw. Kanäle gezeigt. Zum einen kann das Antennenelement die schmale Rechteck- oder Streifenform K aufweisen. Das Antennenelement kann aber auch wie bei K' und K" gezeigt, breit rechteckförmig oder quadratisch oder kreisförmig sein und in der Mitte eine Interventionsöffnung A aufweisen. Gerade die Version mit der Interventionsöffnung eignet sich besonders für den in dieser Anmeldung beschriebenen Anwendungsfall, in welchem die Lokalspulenvorrichtung eine Interventionsöffnung 2 aufweist. So kann zum Beispiel das Antennenelement K' um die Interventionsöffnung der Lokalspulenvorrichtung herum angeordnet sein, das gilt sowohl für die Version mit einem Fenster als auch mit zwei Fenstern.

Auch erlaubt es die Form des Antennenelementes K' das Fenster 2 im ersten Trägerbereich T1 der Lokalspulenvorrichtung mittig anzuordnen, so dass die wenigstens eine Interventionsöffnung 2 - wie z. B. in Figur 1 gezeigt - auf beiden Seiten der Linie T1y und ggf. zu beiden Seiten der Linie T1x liegt. Neben anderen beliebig vorzusehenden Kanälen wird ein Antennenelement vom Typ K' (Figur 11) um die Interventionsöffnung 2 herum angeordnet. Gleiches gilt für die Version mit 2 Interventionsöffnungen, (vgl. z.B. Figur 9A, 9B). Auch hier können die Antennenelemente vom Typ K' (vgl. Figur 11) um die Interventionsöffnungen 2, 2' herum angeordnet werden. Ebenso wie bei nur einer Interventionsöffnung 2 (vgl. Figur 1) ist dann auch die Anordnung der Interventionsöffnungen in der Mitte der Lokalspulenvorrichtung möglich, bei der sich die Interventionsöffnungen beidseits der Linie T1y erstrecken. Oberhalb und unterhalb der mittig angeordneten Interventionsöffnungen 2 bzw. 2' können weitere Reihen mit Kanälen bzw. Antennenelementen vorgesehen sein. Der Anmelder behält sich vor, auf diesen Aspekt ein separates Schutzbegehren, beispielsweise in Form einer Teilanmeldung, zu richten.

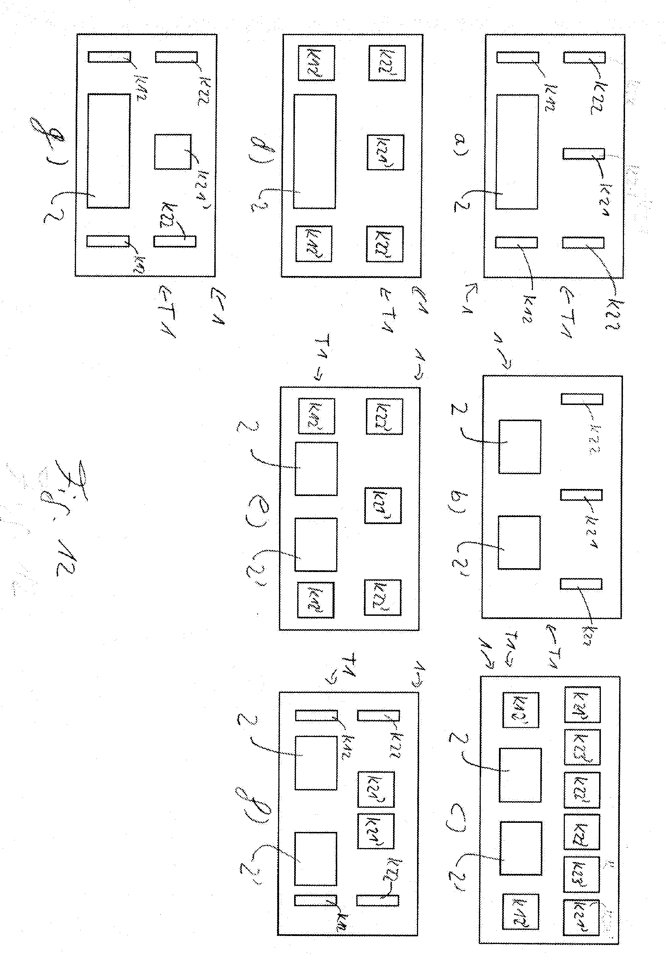

Unter Bezugnahme auf Figur 12 wird eine weitere Ausführungsform der Erfindung erläutert, bei der Antennenelemente unterschiedlicher Breite und/oder breite Antennenelemente verwendet werden. Bei einem breiten Antennenelemente ist die Breite größer als etwa 50 %, vorzugsweise größer als etwa 66 %, mehr bevorzugt größer als etwa 75 % höchst bevorzugt größer als etwa 87,5 % der Länge des Antennenelementes. Ein breiteres Antennenelement kann MRT-Signale aus einem größeren Bereich empfangen. Somit müssen weniger Kanäle von der Lokalspulenvorrichtung zur Auswerteeinrichtung übertragen werden.

Die Lokalspulenvorrichtung 1 gemäß Figur 12a umfasst eine Interventionsöffnung 2. Neben der Interventionsöffnung 2 ist je ein schmales Antennenelement K12 angeordnet. In Querrichtung des ersten Trägerbereichs oben befinden sich drei schmale Antennenelemente K21, K22, die in Längsrichtung des ersten Trägerbereichs T1 im Wesentlichen äquidistant angeordnet sind.

Die Lokalspulenvorrichtung 1 gemäß Figur 12b umfasst zwei Interventionsöffnungen 2 und 2'. In Querrichtung des ersten Trägerbereichs oben befinden sich drei schmale Antennenelemente K21, K22, die in Längsrichtung des ersten Trägerbereichs T1 im Wesentlichen äquidistant angeordnet sind. Es befinden sich jedoch keine Antennenelemente neben den Interventionsöffnungen.

Die Lokalspulenvorrichtung 1 gemäß Figur 12c weist zwei Interventionsöffnungen 2 und 2' auf. Neben der Gruppe von Interventionsöffnungen 2, 2' ist je ein breites Antennenelement K12' angeordnet. In Querrichtung des ersten Trägerbereichs oben befinden sich sechs breite Antennenelemente K21 ', K22', K23', die in Längsrichtung des ersten Trägerbereichs T1 im Wesentlichen äquidistant angeordnet sind.

Die Lokalspulenvorrichtung 1 gemäß Figur 12d weist eine Interventionsöffnung 2 auf. Neben der Interventionsöffnung 2 ist je ein breites Antennenelement K12' angeordnet. In Querrichtung des ersten Trägerbereichs oben sind drei breite Antennenelemente K21 ', K22' angeordnet, die in Längsrichtung des ersten Trägerbereichs T1 im Wesentlichen äquidistant angeordnet sind.

Die Lokalspulenvorrichtung 1 gemäß Figur 12e weist zwei Interventionsöffnungen 2, 2' auf. Neben der Gruppe von Interventionsöffnungen 2, 2' ist je ein breites Antennenelement K12' angeordnet. In Querrichtung des ersten Trägerbereichs T1 oben sind drei breite Antennenelemente K21 ', K22' angeordnet, die in Längsrichtung des ersten Trägerbereichs T1 im Wesentlichen äquidistant angeordnet sind.

Die Lokalspulenvorrichtung 1 gemäß Figur 12f weist zwei Interventionsöffnungen 2, 2' auf. Neben der Gruppe von Interventionsöffnungen 2, 2' ist je ein schmales Antennenelement K12 angeordnet. In Querrichtung des ersten Trägerbereichs T1 oben sind zwei breite Antennenelemente K21' und zwei schmale Antennenelemente K22 angeordnet. Die breiten Antennenelemente K21' sind in Längsrichtung des ersten Trägerbereichs T1 im mittleren Bereich angeordnet. Die schmalen Antennenelemente K22 sind in Längsrichtung des ersten Trägerbereichs T1 betrachtet am Rand angeordnet.

Die Lokalspulenvorrichtung 1 gemäß Figur 12g weist eine Interventionsöffnung 2 auf. Neben der Interventionsöffnung 2 ist je ein schmales Antennenelement K12 angeordnet. In Querrichtung des ersten Trägerbereichs T1 oben sind ein breites Antennenelement K21' und zwei schmale Antennenelemente K22 angeordnet. Das breite Antennenelemente K21' ist in Längsrichtung des ersten Trägerbereichs T1 im mittleren Bereich angeordnet. Die schmalen Antennenelemente K22 sind in Längsrichtung des ersten Trägerbereichs T1 betrachtet am Rand angeordnet.

Bei den Ausführungsformen gemäß Figuren 12a bis 12g kann ein Antennenelement um die Interventionsöffnung 2, 2' oder um eine Mehrzahl von Interventionsöffnungen 2, 2' angeordnet sein.

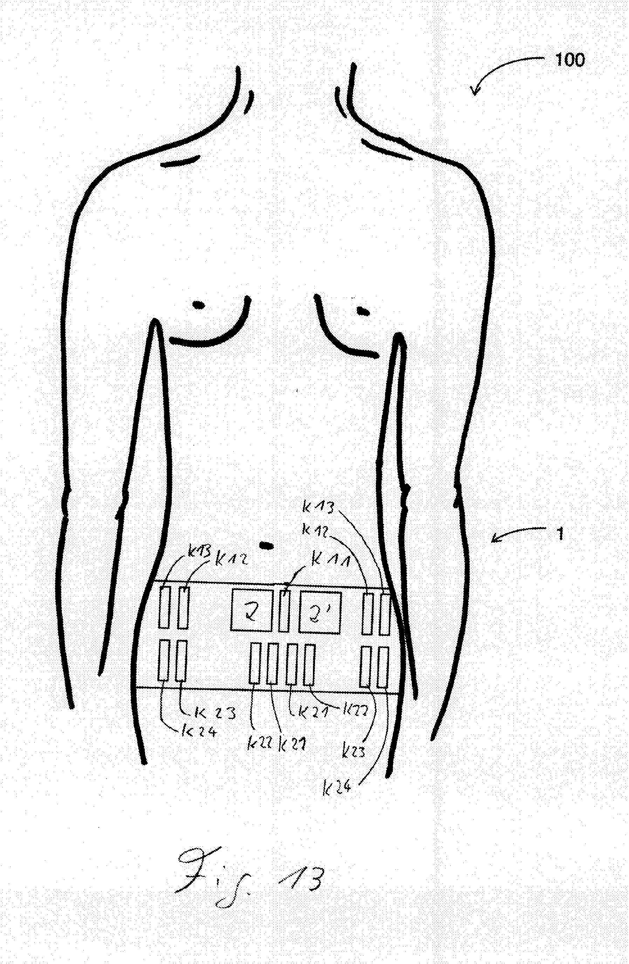

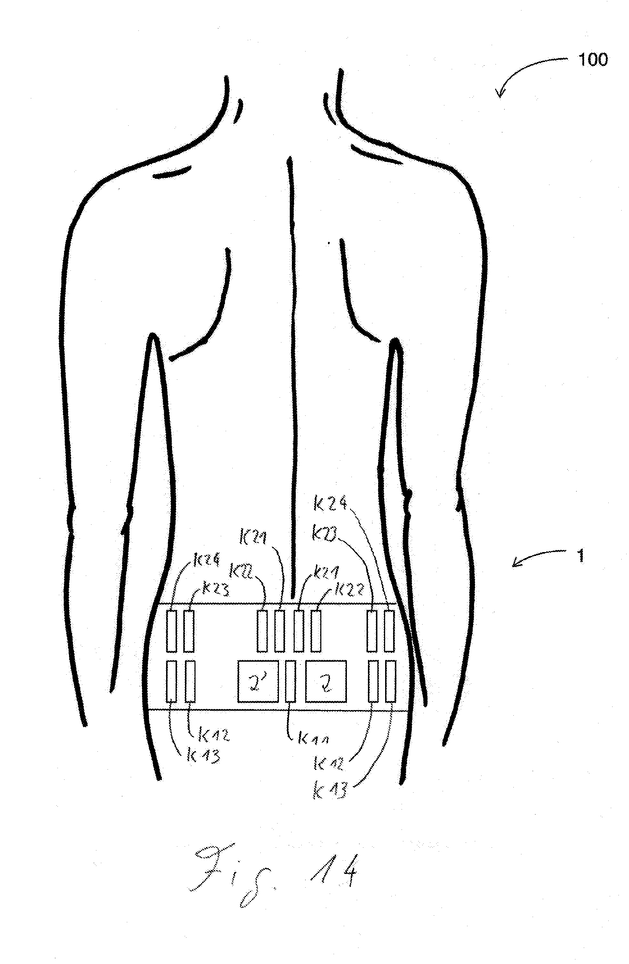

Abschließend wird unter Bezugnahme auf Figuren 13 und 14 eine praktische Anwendung der Erfindung bei der Behandlung der Prostata erläutert, in denen schematisch der Rücken eines Patienten 100 dargestellt ist.

In der in Figur 13 gezeigten Stellung des ersten Trägerbereichs T1 wirkt die MRT-Lokalspulenvorrichtung 1 als Diagnosespulenvorrichtung. Der Patient liegt mit seinem Rücken auf dem zweiten Trägerbereich T2 (nicht gezeigt). Der zweite Trägerbereich T2 kann so ausgebildet sein, wie in den Ausführungsformen gemäß Figuren 9B, 10B und 10C beschrieben wurde. Der erste Trägerbereich befindet sich in der Diagnosestellung. In der Diagnosestellung befinden sich die Antennenelemente K21 und K22 in sagittaler Richtung ventral über dem kleinen Becken bzw. in longitudinaler Richtung des Patienten auf gleicher Höhe wie das kleine Becken. In der Mitte des kleinen Beckens befinden sich bei einer Frau die Eierstöcke, die Gebärmutter und die Harnblase. Bei einem Mann befinden sich in der Mitte des kleinen Beckens die Prostata, die Samenblase und die Harnblase. Folglich kann in der Diagnosestellung eine besonders genaue Bildgebung des kleinen Beckens erfolgen.

In der Diagnosestellung befindet sich das Antennenelement K 11 in einem mittleren Bereich des großen Beckens, in dem sich hauptsächlich Darmschlingen befinden. Die Darmschlingen sind bei der Therapie des Prostatakarzinoms von weniger starker Bedeutung, da dieser Bereich üblicherweise in einem geringeren Ausmaß von Metastasen aufgrund eines Prostatakarzinoms betroffen ist.

Die Antennenelemente K12 und K13 befinden sich am Rand des großen Beckens. Dieser Bereich ist stärker von Metastasen des Prostatakarzinoms oder eines Karzinoms eines anderen Organs betroffen, da dieser Bereich eine große Anzahl von Lymphgefäßen aufweist. Aufgrund des von der Prostata in Richtung Rand des oberen Beckens fließenden venösen Blutes sind die Lymphknoten am Rand des großen Beckens häufiger von Metastasen des Prostatakarzinoms oder eines Karzinoms eines anderen Organs befallen.

In der Diagnosestellung befinden sich die Antennenelemente K12, K13, K21 und K22 über den Körperpartien, die bei der Diagnose des Prostatakarzinoms von besonderer medizinischer Relevanz sind, d.h. über dem kleinen Becken und dem Rand des großen Beckens.

Figur 14 zeigt die Interventionsstellung des ersten Trägerbereichs T1. Der Patient liegt mit seinem Bauch auf dem zweiten Trägerbereich. Die Interventionsöffnungen 2, 2' befinden sich in sagittaler Richtung über dem kleinen Becken. In dieser Stellung ist eine Punktion der Prostata möglich. Da sich das Antennenelement K11 zwischen den Interventionsöffnungen 2, 2' befindet und sich die Antennenelemente K21 und K22 nahe an den Interventionsöffnungen 2, 2' befinden ist immer noch eine vergleichsweise gute Bildgebung des kleinen Beckens möglich.

Es wird wieder auf Figur 10B Bezug genommen, die den zweiten Trägerbereich zeigt. Der Patient liegt auf dem zweiten Trägerbereich T2 (nicht gezeigt) mit dem Bauch. Der zweite Trägerbereich T2 kann so ausgebildet sein, wie in den Ausführungsformen gemäß Figuren 9G, 10B und 10C beschrieben wurde. Die Antennenelemente K200 und K201 befinden sich immer, d.h. sowohl in der Diagnosestellung als auch in der Interventionsstellung des ersten Trägerbereichs, in sagittaler Richtung dorsal unter dem kleinen Becken. Dadurch wird sichergestellt, dass auch in der Interventionsstellung des ersten Trägerbereichs T1 eine zufriedenstellende Bildgebung des kleinen Beckens möglich ist.

In der Diagnosestellung ist die Anzahl der Antennenelemente in sagittaler Richtung ventral über dem kleinen Becken höher als in der Interventionsstellung des ersten Trägerbereichs T1. Der zweite Trägerbereich T2 wird stets so angeordnet, das sich eine höhere Anzahl von Antennenelementen in sagittaler Richtung dorsal unter dem kleinen Becken befindet.

Da der erste Trägerbereich T1 vom zweiten Trägerbereich T2 trennbar ist und gegenüber diesem um 180° geschwenkt werden kann, schafft die Erfindung eine flexible MRT-Spulenvorrichtung, die sowohl zur Diagnose als auch zur Intervention eingesetzt werden kann. Der Anmelder behält sich vor, auf diesen Gegenstand ein separates Schutzbegehren zu richten.

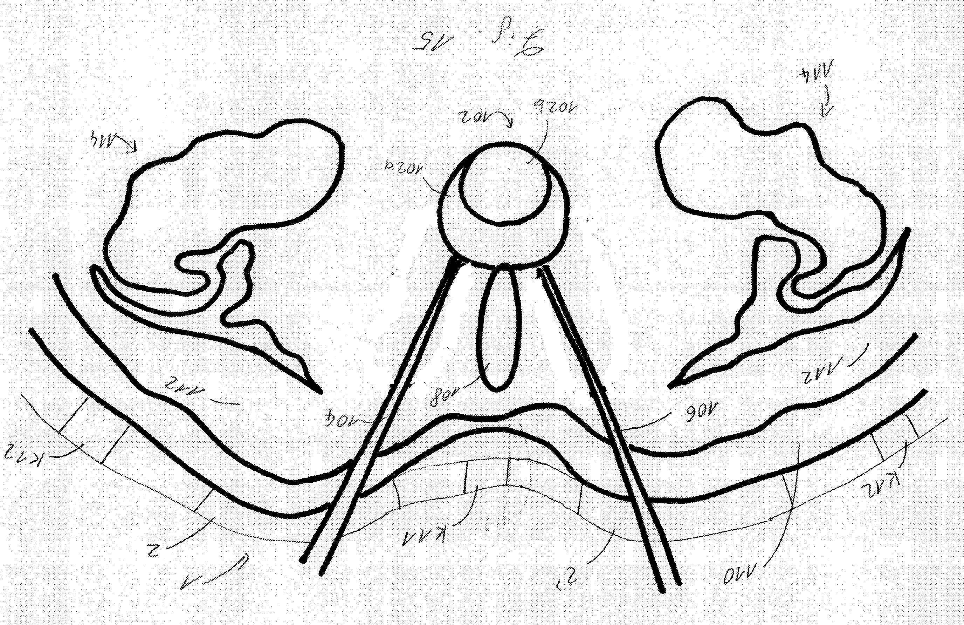

Unter Bezugnahme auf Figur 15 wird die Anwendung der erfindungsgemäßen Lokalspulenvorrichtung 1 bei einer Prostatapunktion erläutert. Figur 15 stellt einen Schnitt entlang der Transversalebene auf der Höhe der Prostata eines Patienten dar. Figur 15 zeigt die Prostata 102, die Hüftgelenke 114, den Dickdarm 108, den Gesäßmuskel 112 und die äußere Fettschicht 110. Die Prostata weist eine periphere Zone 102a und eine transitionale Zone 102b auf. Auf der Haut über der Fettschicht 110 ist die Lokalspulenvorrichtung 1 angeordnet. Die Lokalspulenvorrichtung 1 umfasst zwei Interventionsöffnungen 2, 2', die neben der Pofalte angeordnet sind. Über der Pofalte und zwischen den Interventionsöffnungen 2, 2' befindet sich das schmale Antennenelement K11. An der der Pofalte abgewandten Seite der Interventionsöffnungen 2, 2' befindet sich je das Antennenelement K12.

Das Auswerten der MRT-Antwortsignale, die mittels der Antennenelemente empfangen werden, ermöglicht dem Chirurg eine MRT-gestützte Biopsie durchzuführen. Während Biopsienadeln 104, 106 in das Gesäß in Richtung Prostata geführt werden, kann der Chirurg aufgrund der Bildinformation festlegen, unter welchen Winkel und bis zu welcher Tiefe die Biopsienadeln einzuführen sind, damit eine Gewebeprobe aus einem verdächtigen Bereich der Prostata entnommen werden kann. Während des Einführenes der Biopsienadeln können sukzessive MRT-Bildaufnahmen erstellt werden, um die aktuelle Position der Biopsienadeln zu erfassen, bis die Biopsienadeln das zu untersuchende Gewebe erreicht haben.

Das Verfahren kann manuell durchgeführt werden. Es ist auch möglich, dass die Biopsienadeln auf Grundlage von MRT-Bildaufnahmen automatisiert zu dem zu untersuchenden Gewebe geführt werden.

Die Erfindung ermöglicht, dass eine Biopsie der Prostata durchgeführt werden kann, ohne dass der Dickdarm verletzt wird. Die Erfindung kann verhindern, dass während der Biopsie Bakterien aus dem Dickdarm in die Prostata gelangen. Ferner ermöglicht die vorliegende Erfindung, dass die Biopsie unter Anästhesie durchgeführt werden kann.

Die erfindungsgemäße Lokalspulenvorrichtung kann zum Diagnostizieren und/oder zur Intervention anderer Organe verwendet werden, beispielsweise der Niere, der Galle, der Bauchspeicheldrüse etc. Der Patient muss während der Diagnose und/oder während der Intervention nicht notwendigerweise auf dem Rücken bzw. Bauch liegen. Die Interventionsöffnung kann sich beispielsweise bei der Diagnose und/oder während der Intervention der Bauchspeicheldrüse auf dem Bauch befinden. Ferner kann sich die Interventionsöffnung beispielsweise bei der Diagnose und/oder während der Intervention der Galle seitlich am Körper befinden. Die Interventionsöffnung befindet sich dort, wo der medizinisch beste Zugang zum Organ möglich ist.

Die mittels der erfindungsgemäßen Lokalspulenvorrichtung erzeugten Bilddaten können an ein Operationsnavigationssystem übergeben werden, damit der Verlauf des Eingriffs und das Positionieren des Instrumentes durch das Operationsnavigationssystem genauer assistiert werden kann.

Generell gilt für alle Ausführungsformen, dass logische Kanäle Signale von wenigstens einem Antennenelement verarbeiten, jedoch auch Signale von einer Mehrzahl Antennenelemente, die hier nicht näher dargestellt sind, bündeln.

Alle Ausführungsformen der Erfindung lassen sich auch miteinander kombinieren. Die Erfindung ist daher nicht auf die in den Zeichnungen dargestellten Ausführungsformen beschränkt, sondern umfasst auch alles, was sich durch Zusammenführen von Elementen einzelner Ausführungsformen noch konstruieren lässt.

Insbesondere können die Ausführungsformen gemäß Figuren 1 bis 7 mit den Ausführungsformen gemäß Figuren 8 bis 15 kombiniert werden. Mit anderen Worten, an der Interventionsöffnung 2 bzw. an den Interventionsöffnungen 2, 2' gemäß Figuren 8 bis 15 können ein Rahmen, eine Traverse, erste und zweite Justagemittel, Markierungen, eine Skala ein Referenzelement, ein Schlitten, ein Instrumenthalter, ein Nadelhalter, ein Instrument, eine Nadel etc. angeordnet sein, wie unter Bezugnahme auf die Ausführungsformen gemäß Figuren 1 bis 7 beschrieben ist.

Im Kontext dieser Erfindung ist die sagittale Richtung die Richtung vom Rücken zum Bauch oder umgekehrt. Im Kontext dieser Erfindung ist die Logitudinalrichtung die Richtung vom Kopf zum Fuß oder umgekehrt. Im Kontext dieser Erfindung ist die Längsrichtung des Trägers länger als dessen Querrichtung.

## Patentansprüche

1. MRT-Lokalspulenvorrichtung (1), mit
- einem Träger (1a, 1 b; T1, T2); und
- einer Mehrzahl von Antennenelementen (K11-K202), die je zum Empfangen von MRT-Signalen ausgebildet sind;
wobei die Mehrzahl von Antennenelementen (K, K11-K202) in dem Träger (1a, 1 b; T1, T2) angeordnet ist;
**dadurch gekennzeichnet, dass** der Träger (1a, 1 b; T1) zumindest eine Interventionsöffnung (2) aufweist, die dazu ausgebildet ist, einen Zugang für ein medizinisches Instrument zum Patienten bereitzustellen.

2. MRT-Lokalspulenvorrichtung nach Anspruch 1, **dadurch gekennzeichnet, dass** eine höhere Anzahl von Antennenelementen (K21-K24) in einem Bereich angeordnet ist, der sich nahe an einem zu diagnostizierenden Organ befindet, wobei optional eine höhere Anzahl von Antennenelementen (K21-K24) in einem Bereich angeordnet ist, der sich nahe an der Mitte des kleinen Beckens und/oder der Lateralwand des großen Beckens befindet.

3. MRT-Lokalspulenvorrichtung nach Anspruch 1 oder 2, **dadurch gekennzeichnet, dass** der Träger (1a, 1 b; T1, T2) als Manschette ausgebildet ist, die schließbar und öffenbar ist.

4. MRT-Lokalspulenvorrichtung (1) nach einem der vorangegangenen Ansprüche, **dadurch gekennzeichnet, dass** die Interventionsöffnung (2, 2') zur Aufnahme eines in einem MRT-Bild sichtbaren Referenzelements (6) mit ersten Justagemittel ausgebildet ist.

5. MRT-Lokalspulenvorrichtung (1) nach einem der vorangegangenen Ansprüchen, **dadurch gekennzeichnet, dass** die Interventionsöffnung (2, 2') durch einen Rahmen (5, 5') begrenzt wird, wobei an zumindest zwei nebeneinander liegenden Seiten des Rahmens geneigte Gleitflächen (5a, 5b) vorgesehen sind, wobei die Gleitflächen (5a, 5b) gegenüber einer zur Interventionsöffnung (2, 2') senkrechten Achse in die gleiche Richtung geneigt sind.

6. MRT-Lokalspulenvorrichtung (1) nach einem der vorangegangenen Ansprüche, **gekennzeichnet durch** Anordnungsmittel (5c, 5d) für ein medizinisches Instrument (9).

7. MRT-Lokalspulenvorrichtung (1) nach einem der vorangegangenen Ansprüche, **gekennzeichnet durch** Traversenanordnungsmittel (5c, 5d), die zum Anordnen einer in wenigstens einer ersten Richtung (Y) verschiebbaren Traverse (7) über der Interventionsöffnung (2, 2') vorgesehen ist, wobei mit der Traverse (7) optional ein Schlitten (8a) gekoppelt ist, der in eine zweiten Richtung (X) beweglich ist.

8. MRT-Lokalspulenvorrichtung (1) nach einem der vorangegangenen Ansprüche, **dadurch gekennzeichnet, dass** an der Interventionsöffnung (2), an der Traverse (7) und/oder am Schlitten (8) ein medizinisches Instrument, beispielsweise eine Nadel (9), anordenbar ist.

9. MRT-Lokalspulenvorrichtung (1) nach einem der vorangegangenen Ansprüche, **dadurch gekennzeichnet, dass** die Traverse, der Schlitten (8), eine Instrumentenhalterung und/oder der Randbereich der Interventionsöffnung (2, 2') zweite Justagemittel (7e) aufweist.

10. MRT-Lokalspulenvorrichtung (1) nach einem der vorangegangenen Ansprüche, **dadurch gekennzeichnet, dass** zumindest ein Antennenelement (K11) zwischen zwei Interventionsöffnungen (2, 2') angeordnet ist und/oder dass die Wicklung eines Antennenelementes (K', K") um die Interventionsöffnung (A) angeordnet ist.

11. MRT-Lokalspulenvorrichtung (1) nach einem der vorangegangenen Ansprüche, **dadurch gekennzeichnet, dass** die Wicklung des Antennenelements (K11-K202) im Wesentlichen rechteckig ausgebildet ist und/oder zumindest ein Antennenelement (K11-K202) rechtwinklig oder parallel zu einer Kante der Interventionsöffnung (2, 2') angeordnet ist.

12. MRT-Lokalspulenvorrichtung (1) nach einem der vorangegangen Ansprüche, **dadurch gekennzeichnet, dass** sich der Träger (1a, 1 b) in Längsrichtung (T1y) weiter erstreckt als in Querrichtung (T1x) und zumindest ein Antennenelement (K21, K22) in Querrichtung des Trägers (1a, 1 b) neben der Interventionsöffnung (2, 2') und zumindest ein Antennenelement (K11) in Längsrichtung des Trägers (1a, 1b) neben dem Antennenelement befindet.

13. MRT-Lokalspulenvorrichtung (1) nach Anspruch 12, **dadurch gekennzeichnet**, zumindest drei Antennenelemente (K12, K22) in Querrichtung des Trägers (1, 1') neben der zumindest einen Interventionsöffnung (2, 2) angeordnet sind.

14. MRT-Lokalspulenvorrichtung (1) nach einem der vorherigen Ansprüche, **dadurch gekennzeichnet, dass** der Träger (1, 1') einen ersten Trägerbereich (T1), der dazu ausgebildet ist, an einer ersten Seite einer Körperpartie eines Patienten angeordnet zu werden, und einen zweiten Trägerbereich (T2) aufweist, der dazu ausgebildet ist, an einer zweiten Seite der Körperpartie angeordnet zu werden, wobei die zumindest eine Interventionsöffnung (2, 2') im ersten Trägerbereich (T1) ausgebildet ist und wobei sowohl im ersten Trägerbereich (T1) als auch im zweiten Trägerbereich (T2) Antennenelemente (K, K11-K203) angeordnet sind.

15. MRT-Lokalspulenvorrichtung (1) nach Anspruch 14, **dadurch gekennzeichnet, dass** der erste Trägerbereich (T1) in zwei unterschiedlichen Stellungen gegenüber dem zweiten Trägerbereich (T2) anordenbar ist.

16. MRT-Lokalspulenvorrichtung (1) nach Anspruch 14 oder 15, **dadurch gekennzeichnet, dass** der erste Trägerbereich (T1) gegenüber dem zweiten Trägerbereich (T2) so beweglich ist, dass die Interventionsöffnung (2) an zwei unterschiedlichen Stellungen anordenbar ist.

17. MRT-Lokalspulenvorrichtung (1) nach einem der Ansprüche 14 bis 16, **dadurch gekennzeichnet, dass** der erste Trägerbereich (T1) gegenüber dem zweiten Trägerbereich (T2) so anordenbar ist, dass die Interventionsöffnung (2) an zwei unterschiedlichen Stellungen in Querrichtung der der MRT-Lokalspulenvorrichtung (1) und/oder in Longitudinalrichtung des Patienten anordenbar ist

18. MRT-Lokalspulenvorrichtung (1) nach einem der Ansprüche 14 bis 17, **dadurch gekennzeichnet, dass** die MRT-Lokalspulenvorrichtung (1) am Becken oder Bauch eines Patienten (100) anordenbar ist, wobei der erste Trägerbereich (T1) dazu ausgebildet ist, in der Diagnosestellung vorne am Patienten (100) angeordnet zu werden, und der zweite Trägerbereich (T2) dazu ausgebildet ist, in der Diagnosestellung am Rücken des Patienten angeordnet zu werden, wobei der erste Trägerbereich (T1) dazu ausgebildet ist, in der Interventionsstellung am Rücken des Patienten (100) angeordnet zu werden, und der zweite Trägerbereich (T2) dazu ausgebildet ist, in der Interventionsstellung vorne am Patienten angeordnet zu werden, und wobei der erste Trägerbereich (T1) so gegenüber dem zweiten Trägerbereich (T2) anordenbar ist, dass die Interventionsöffnung (2) an zwei unterschiedlichen Stellungen in der longitudinalen Richtung des Patienten anordenbar ist.

19. Medizinisches System mit,
- einer MRT-Lokalspulenvorrichtung (1) nach einem der Ansprüche 1 bis 18;
- einem medizinischen Instrument, das über der zumindest einen Interventionsöffnung (2, 2') anordenbar ist; und
- einem Magnetresonanzsystem, mit dem die MRT-Lokalspulenvorrichtung (1) gekoppelt ist.

20. Verfahren zum Umwandeln einer MRT-Lokalspulenvorrichtung (1) in eine Diagnose-Lokalspulenvorrichtung oder eine Interventions-Lokalspulenvorrichtung und umgekehrt, **gekennzeichnet durch**
- Bewegen eines ersten Trägerbereichs (T1) mit Antennenelementen (K11-K24) gegenüber einem zweiten Trägerbereich (T2) mit Antennenelementen (K, K200-K203).
